# EUROPEAN PATENT APPLICATION

(11) **EP 4 316 589 A2**
(43) Date of publication of application: **07.02.2024**
(21) Application number: 23200414.3
(22) Date of filing: 06.02.2019
(51) Int. Cl.: A61P 13/12

(54) **TREATMENT OF PATIENTS WITH CLASSIC FABRY DISEASE**

(30) Priority: 06.02.2018 US 201862626992 P
(62) Divisional of application: 19706182.3
(71) Applicant: Amicus Therapeutics, Inc., Philadelphia, PA 19104 (US)
(72) Inventor: Barth, Jay, Cranbury, New Jersey, 08512 (US); Benjamin, Elfrida, Cranbury, New Jersey, 08512 (US)
(74) Representative: Hoffmann Eitle

(57) **Abstract**

Provided are methods for the treatment of classic Fabry disease in a patient. Certain methods comprise administering to the patient about 123 mg free base equivalent of migalastat for reducing kidney globotriaosylceramide, stabilizing renal function, reducing left ventricular mass, reducing plasma globotriaosylsphingosine and/or treating gastrointestinal symptoms.

SEQUENCE LISTING

<110>  AMICUS THERAPEUTICS, INC.

<120>  TREATMENT OF PATIENTS WITH CLASSIC FABRY DISEASE

<130>  AT18-001

<150>  US 62/626,992
<151>  2018-02-06

<160>  3

<170>  PatentIn version 3.5

<210>  1
<211>  12436
<212>  DNA
<213>  Homo sapiens

<400>  1
   cccttctgta ggggcagaga ggttctactt cattactgcg tctcctggga aggccatcag      60
   gactgctggc taaagtggga accaggactc tttgtgagtt aagaatttgt gtatttatat     120
   gtgtgttata cacatttttt aaaaaactgt aacgacatca ggttgagcag tcgtctccgg     180
   gtggtgaatt atgtgtattt ttaaatttta tactatattg ttatttttca aatgttcgaa     240
   attgaatatg tagattgttg ttatcagcag aaaaataaac attattcaaa tactctattc     300
   agtaaagtaa tttattgggc gcctttgtca agcacgcatt tgcctagatg tgactctaca     360
   gataaaattc acttggggcc tccccttaca gacaatcagg cagtggagac tgagtgcctg     420
   aatggataga ccagcactca gaccactatt ttcagtatct gtttttctta actcagggcc     480
   gtggttttca aacgtttttc gccttacggt cacccttagg gtcccccgag accggcccag     540
   acagacagat atacaaaaac acatacacag tcatgagcgt ccaccatttc cccaccaggc     600
   gcagcacagg cggcttcccg gcactgagat gggggggagg agggagagag cgcgaggggg     660
   gaggggaaag cagagaacga aagaggcgga ggcggccccc gaaccccgct ctggtcttca     720
   tcatcaccac ccctgggtcc ccagttccca cccacacacc aacctctaac gataccgggt     780
   aattttcctc cttcttccct caaacggcta tagcgagacg gtagacgacg accagaacta     840
   cttctgctca cgtaagcgag taatcacgtg agcgcctacg tcatgtgaga tctcggtcac     900
   gtgagcaact ctcggcttaa actcgggatc actaaggtgc cgcacttcct tctggtatgg     960
   aaatagggcg ggtcaatatc aagaaaggaa gagggtgatt ggttagcgga acgtcttacg    1020
   tgactgatta ttggtctacc tctggggata accgtcccag ttgccagaga aacaataacg    1080
   tcattattta ataagtcatc ggtgattggt ccgcccctga ggttaatctt aaaagcccag    1140
   gttacccgcg gaaatttatg ctgtccggtc accgtgacaa tgcagctgag gaacccagaa    1200
   ctacatctgg gctgcgcgct tgcgcttcgc ttcctggccc tcgtttcctg ggacatccct    1260
   ggggctagag cactggacaa tggattggca aggacgccta ccatgggctg gctgcactgg 1320
   gagcgcttca tgtgcaacct tgactgccag gaagagccag attcctgcat caggtatcag 1380
   atattgggta ctcccttccc tttgcttttc catgtgtttg ggtgtgtttg gggaactgga 1440
   gagtctcaac gggaacagtt gagcccgagg gagagctccc ccacccgact ctgctgctgc 1500
   ttttttatcc ccagcaaact gtcccgaatc aggactagcc ctaaactttc tctgtgtgac 1560
   ctttcctggg atgggagtcc ggccagcggc ccctgtttct ttctctctct ctctctctct 1620
   cgttctcctt ctctttctct ttctcttctt tcctctctct ttctctctct ccctgcccgg 1680
   ttctcttttt tcactgctcc ttgcagagca gggccacccc ataggcagtg tgcccaaagt 1740
   agccctgccc ggttctattc agacccttct tgtgaacttc tgctcttcct ctgccgggtg 1800
   ctaaccgtta gaacatctag ggtgggtagg aggaatgggg aactaagatt cgtgccattt 1860
   tttctccttt tggggtcgtg gatttctcgg cagtatctcg agggagttag agagaccata 1920
   aggtcgctga gatctctccc acctcgccca tgagcgtggc atcaggctgg aaggttgaca 1980
   tggaggaact ttatacattt acacctttgc gtgagggttg aggctggatt agataggtat 2040
   tgaacatatc tgaccctcac aatccttatc tgtaaattgg gattacaacc ttttaatttc 2100
   agggagctga caaaaaaaat ctgaaaaata gttcttatct cacacaggtg agttttcaag 2160
   gagataacct atttaaagta catagcacag cgcttgacca ttcaactgcg cttacagagc 2220
   aaatgttcaa tgggaaaatg aatgtaaatc tacaaatctg aatgaatatg tgtatttttc 2280
   tggagagagg atatttacct ttcttcaaat tctcaaaggg ctctgtgatt taaaaaaggt 2340
   taggaatcac tgatagatgt tggtaaaagg tggcagtcac agtacatttc tgtgtccata 2400
   agttattcct atgaatatct ttatagataa agtcaggatg ttggtcagac atcacagaag 2460
   aaattggcct tgtaagtttc atgtgaccct gtggtacagt atgtgtggca attttgccca 2520
   tcacggattt ttttttattg gtatttgcat ctgattataa aactaatgca tgatcattgc 2580
   aaaaaatgta gataaagaag agcaaaatga aaataaagat ttccccccac cgttccacca 2640
   cccagaaata atcatggttt aaatgttaat atacaacctt acaattgttt tctatataaa 2700
   tgaaaacata gatttcttta tttcattatt ttccataaaa aatggatcat gtttatgtca 2760
   tgtttggcta atggcaagac cctggcaccc agtctgggct caaattctgc ctcattgtta 2820
   cttagccctg tgacattggg taaattacac tttttttttt tttttttttt tgagacgggg 2880
   tctcgctctg tcgcccaggc tggagtgcag tggcacgatc tcggctcact gcaagtccgc 2940
   ctcctgggtt cacgccattc ttctgcctca gcctcccgag tagctgggac tacaggcgcc 3000
   tgccaccacg cctggctctt tttttttttt tttttttttt tagtacagac ggggtttcac 3060
   catgttagcc agggtggtct caatctcctg acctcgtgat tcgcccgcct cagcctccca 3120
   aagtgctggt gtgagccacc gtgcccagcc ttactttttt ttttgagagg gggtctcact 3180
   ctgtcaccca ggttggagtg cagtggcgcg atctctgctc agtgcaaact ccacctcccg 3240
   ggtttaagca gttctcctgt cgtagtctcc tgagtagctg ggattacagg cacaccacca 3300
   cggccagcta atttttgtat tttcagtaga gacgggtttc accatgttgc ccaagctggt 3360
   ctcgaactcc tggcctcaag tgatctgccc gccttggcct cccagagtgc tgggattaca 3420
   ggtgtgagcc accgcacccg gcctcttttt tcttttttag tctatcatac cttgcaaata 3480
   cagtggttct tcctatgtgt tggttttgat atttatgtaa tcaaacacat cagtttttcc 3540
   tttctgattt ctgactttgg ggtcatgctg agaaagtcct ttcctacctg aagataatac 3600
   agtatatacg tttcttacta gtatttttgt ggatttttaa aatatttaaa tctttagtcc 3660
   atctgaactt gttcttctat cagaaatgcc acatttaata aataataagt cccatggtat 3720
   cagatggctg gaaggacctc tttcgaaact ttgtttaatt ccattaatct gtgtattctt 3780
   attctaatgc taatagttcc acactagctt cctttatctt tttttctttt tttttttttt 3840
   ttttgagctg gagtttcgct cttgttgccc aggctggagt acaatgtcac gatctcggtt 3900
   caccgcaacc tccgcctccc aggttcaagc aattctcctg cctcatcctc gcgagtagct 3960
   ggaattacag gcatgcgcca ccacgcctag ctattttgta tttttagtag agatggggtt 4020
   tctccatgtt ggtcaggctg gtctcaaact cccagcctca ggtgatctgc ctgcctcggc 4080
   ctcccaaaat gctgttatta caggcgtgag ccaccacgcc cagccttcat cttttaatga 4140
   atgtacatgt atgtaatctt ttaggtgaac tttttgtaat gttgtgccaa gttccttaaa 4200
   aagccctttt ggaagctggg caggtggcca cgcctgtaat cccagcattt tgggagtctg 4260
   aggcaggtgg atcacttgag gccaggagtt caagactagc ctagccaaaa tgcaaaaccc 4320
   tgtctctact aaagatacaa aaattagccg gatgcgatgg cacatgcctg taatctcagc 4380
   tactcgggag gctgaggtag aagaatcgct tgaaccgggg aggcagaggt tgcagtgagc 4440
   aagatggcgc cactgcactc cagcctgggt gacagaggga gactccatct caaaaaaaaa 4500
   aaaaaaaaaa aagataaaaa ggaaacctaa gtactcttgg gctttgttaa ggattttgtt 4560
   aaatatacaa aggattgcag ggaaaattaa cttattttta atattgagta tgcttatcca 4620
   agagcaaaat aatatttctc catttattca aatcatttag gagcatcata gttttaacat 4680
   atgggccttg cacgtatctt aaatttatct ctaggcattt taggttgttc agttgttctt 4740
   gtgaatggga tctttttctc caaataggat tattgttgat atctgttgat tatgttaact 4800
   ttgtagtttc tgactttact gaactgtctt cttagatcta atactctttt caatttcatc 4860
   atatatttct cattcctatt ttgtttgggg tttttagggc gggaatatta acgggataag 4920
   agagacaaaa gaaaatctgg aaaaacaatt catttacctt tacattgctt gtgattacta 4980
   ccacactatt actgggttgg aaaaaattgt gaaatcccaa ggtgcctaat aaatggaggg 5040
   tacctaagtg ttcatttaat gaattgtaat gattattgga atttctcttt cagtgagaag 5100
   ctcttcatgg agatggcaga gctcatggtc tcagaaggct ggaaggatgc aggttatgag 5160
   tacctctgca ttgatgactg ttggatggct ccccaaagag attcagaagg cagacttcag 5220
   gcagaccctc agcgctttcc tcatgggatt cgccagctag ctaattatgt gagtttatag 5280
   ataatgttct tgttcattca gaggactgta agcacttctg tacagaagct tgtttagaaa 5340
   cagccctcat ggccgggcgt ggtggctcac gctgtaatcc caacactttg ggaggccgag 5400
   gcgggtggat cacctgaggt caagagttca agaccagcct ggccaacatg gtgaaacccc 5460
   aactctatta aaagtacaaa aaattagctg ggcatggtgg tgaacgcctg taaccccagc 5520
   tacttgggag gctgaggcag gagaatcgct tgaacccagg aggtggaagt ttcagtgagc 5580
   tgagatcacg ccattgcact ctagcctggg caacaaaaga gaaactccat ctcaaaaaaa 5640
   aaaacaagga aaaaaagaaa cagccctcat gacacttaga aagtagaata gctggctgtt 5700
   atctgaacat tgaattgtaa ggcttatcag gtggactttg cattccatca gcagacaatt 5760
   tttttttttt tttttttttg agatggagtc tcattctgtc tcccaggctg gagggcagtg 5820
   gtgcgatctc ggctcactgc aagctccacc tcctgggttc atgccattct cctgcctcag 5880
   cctcccaagt agctgggacc acaggcaccc gccaccatgc ccagttaatt ttttgtattt 5940
   ttagtagaga cggggtttca ccatgttagc caagatggtc tcgatctcct gacctcgtga 6000
   tccgcccacc tcggcctccc aaagtgctgg gattacaggc atgagccacc gcgcctagcc 6060
   tacaaatgtt ttgtaatagc tcttgaggcc catcttggag ttctcctttt gctaaaacca 6120
   ctgaactctc taggaggaaa aaggaacttg gttcttgaca tatgtgtgca tgtatttcca 6180
   tataaccttt aggaagctat tgcaatggta ctataaacta gaattttaga agatagaagg 6240
   aaaatattct ggagatcatt gaagagaaat ggagtccaac actagttaaa gatgatgaag 6300
   acagattttt ttttttgacg gagtctcgct ctgtcgccca ggctggagtg cagtggcaca 6360
   atctcagctc actgcaaccc tccacctctt gggttcaagt gattctcctg cctcagcctc 6420
   ccaagtagct gggactacag gcgcacacca ccacgcccgg ctaatttttg tatttttagt 6480
   agagacaagg tttcaccata ttcgccaggc tggtctcgaa ctcctgacct tgtaatccgc 6540
   ccaccttggc ctcccaaagt gctgggatta caggcatgag ccaccacgcc cggccgatga 6600
   agacagattt tattcagtac taccacagta gaggaaagag ccaagttcaa ttccaaatac 6660
   aacaaagaca ggtggagatt tatagccaat gagcagattg agggggtcag tggatggaat 6720
   atttaagaag acatcaaggg tagggagctt cttgctaaag cttcatgtac ttaaacaaga 6780
   agggtggggg atgagggaaa ttgatcagat atcaatggtg gcagtattga cttagcagga 6840
   ttcttgctaa gaggtcttgc taggacagac ataggaagcc aaggtggagg tctagtcgaa 6900
   aagaaggctc atcagagaag tctaactaaa gtttggtcaa gaagagtctt tgtcaaggta 6960
   aatctatcat ttccctcaaa aggtaatttt caggatccca tcaggaagat tagcatggct 7020
   gctagctttc tcctcagttc tgggctatag ctcacatgcc tagtttgaac tagctcagca 7080
   gaactggggg atttattctt tgtcttccaa caaactcatc tggatgattt tgggggtttg 7140
   tggggaaaag cccccaatac ctggtgaagt aaccttgtct cttcccccag cctggaatgg 7200
   ttctctcttt ctgctacctc acgattgtgc ttctacaatg gtgactcttt tcctccctct 7260
   catttcaggt tcacagcaaa ggactgaagc tagggattta tgcagatgtt ggaaataaaa 7320
   cctgcgcagg cttccctggg agttttggat actacgacat tgatgcccag acctttgctg 7380
   actggggagt agatctgcta aaatttgatg gttgttactg tgacagtttg gaaaatttgg 7440
   cagatggtaa tgtttcattc cagagattta gccacaaagg aaagaacttt gaggccatgg 7500
   tagctgagcc aaagaaccaa tcttcagaat tttaaatacc ctgtcacaat actggaaata 7560
   attattctcc atgtgccaga gctcccatct cttctctttc agttcattaa ttaattaatt 7620
   aattcatgta aaatccatgc atacctaacc atagctaata ttgtgcactt ataattcaag 7680
   agggctctaa gagttaatta gtaattgtaa ctctctataa catcatttag gggagtccag 7740
   gttgtcaatc ggtcacagag aaagaagcat cttcattcct gcctttcctc aatatacaca 7800
   ccatctctgc actacttcct cagaacaatc ccagcagtct gggaggtact ttacacaatt 7860
   taagcacaga gcaactgcct gtccctgctg ctagtttaaa catgaacctt ccaggtagcc 7920
   tcttcttaaa atatacagcc ccagctgggc atgatggctc atgcctgtaa tcctagcact 7980
   ttgggaggct gaggcgggtg gattacttga ggtcaggagt tcgagaccac cctggccaac 8040
   atggtgaaac cccatctcta gtaaaaatac aaaaattagc tgactttggt ggcacatgcc 8100
   tgtaatccca gctacttggg aagctgagac agaagagtca cttgaacctg ggaaacagag 8160
   gttgcagtga gccaagatcg caccactgca ctccaccctg gatgacagac tgaaccccat 8220
   ctcaaaaaat taaaataaaa taaaataaaa taactatata tatagcccca gctggaaatt 8280
   catttctttc ccttatttta cccattgttt tctcatacag gttataagca catgtccttg 8340
   gccctgaata ggactggcag aagcattgtg tactcctgtg agtggcctct ttatatgtgg 8400
   ccctttcaaa aggtgagata gtgagcccag aatccaatag aactgtactg atagatagaa 8460
   cttgacaaca aaggaaacca aggtctcctt caaagtccaa cgttacttac tatcatccta 8520
   ccatctctcc caggttccaa ccacttctca ccatccccac tgctgtaatt atagcctaag 8580
   ctaccatcac ctggaaagtc atccttgtgt cttccccttt atttcaccat tcatgtcctg 8640
   tctatcaaca gtccttccac cagtatctct aaaatatctc ctgaatcagc ccacttcctt 8700
   ccatcttcac tacatgcacc ctggccttcc aagctactat cggctctcaa ccagactgct 8760
   gggaccacct gatctctctg cttccactct gtctcaaccc ccatctattt tccaagcagc 8820
   actagagtta tcatattaaa atgtaaatat cagttttttt tttaaagaaa aaaaccctga 8880
   gacttaacag agttataaaa aatataaatg tcatcatcag ttccctgctt aaaaccctta 8940
   actcgcttcc aattgcactt ggaatgaaac caaactgcac tgatccagcc cttgcctgcc 9000
   tccccaaagt ccaaggggtc atggctcttt ccctggctac actggttttc tttctgtccc 9060
   tcaacactgc aagcctattg ctgccccagg gcctttacac ttgctttttt tctgcctaga 9120
   acagttcttc cccaaagatt tttaaagggc cgggctcctt aacattgaag tcgcagacca 9180
   aacgccacat atgcagacag ttcttctcta actactttaa aatagccctc tgtccattca 9240
   ttcttcatca cattaacctg tttaattttc ttctcagagc tccacactat ttggaagtat 9300
   ttgttgactt gttaccatgt ctccccacta gagtgtaagt ttcatgaggg caggaccttt 9360
   gtctgacttt gactgtatct ctcgcatatg gttaagtgtt aaatagttat ttatggaatg 9420
   aatccctatt attccctcat tatctctgca aaatagtctt ttttctcaac atcttaaacc 9480
   tgatatccca cctgcctatc tacaaacttt ttttttgcga cagagtctca ctgtcaccca 9540
   ggctagagtg cagtggcgcc atctcggctc actgcaacct ccgcctcccg ggtttaagcg 9600
   attctcttgc ctcagcctcc cagtagctgg gattataggc gtgcgctacc acatctggct 9660
   aatttttgta tttttagtag agatggtttc accatgttgg ccaggcttgt ctcgaactcc 9720
   tgacctcaga tgatccacct gcctcggcct cccaaagtgc tgggattaca ggcatgagcc 9780
   accgtgccca gcctctacaa actttttatt ccattaacaa actatatgct gggatttaag 9840
   ttttcttaat acttgatgga gtcctatgta attttcgagc ttttaatttt actaagacca 9900
   ttttagttct gattatagaa gtaaattaac tttaagggat ttcaagttat atggcctact 9960
   tctgaagcaa acttcttaca gtgaaaattc attataaggg tttagacctc cttatggaga 10020
   cgttcaatct gtaaactcaa gagaaggcta caagtgcctc ctttaaactg ttttcatctc 10080
   acaaggatgt tagtagaaag taaacagaag agtcatatct gttttcacag cccaattata 10140
   cagaaatccg acagtactgc aatcactggc gaaattttgc tgacattgat gattcctgga 10200
   aaagtataaa gagtatcttg gactggacat cttttaacca ggagagaatt gttgatgttg 10260
   ctggaccagg gggttggaat gacccagata tggtaaaaac ttgagccctc cttgttcaag 10320
   accctgcggt aggcttgttt cctattttga cattcaaggt aaatacaggt aaagttcctg 10380
   ggaggaggct ttatgtgaga gtacttagag caggatgctg tggaaagtgg tttctccata 10440
   tgggtcatct aggtaacttt aagaatgttt cctcctctct tgtttgaatt atttcattct 10500
   ttttctcagt tagtgattgg caactttggc ctcagctgga atcagcaagt aactcagatg 10560
   gccctctggg ctatcatggc tgctccttta ttcatgtcta atgacctccg acacatcagc 10620
   cctcaagcca aagctctcct tcaggataag gacgtaattg ccatcaatca ggaccccttg 10680
   ggcaagcaag ggtaccagct tagacaggta aataagagta tatattttaa gatggcttta 10740
   tatacccaat accaactttg tcttgggcct aaatctattt ttttcccttg ctcttgatgt 10800
   tactatcagt aataaagctt cttgctagaa acattacttt atttccaaaa taatgctaca 10860
   ggatcatttt aatttttcct acaagtgctt gatagttctg acattaagaa tgaatgccaa 10920
   actaacaggg ccacttatca ctagttgcta agcaaccaca ctttcttggt ttttcaggga 10980
   gacaactttg aagtgtggga acgacctctc tcaggcttag cctgggctgt agctatgata 11040
   aaccggcagg agattggtgg acctcgctct tataccatcg cagttgcttc cctgggtaaa 11100
   ggagtggcct gtaatcctgc ctgcttcatc acacagctcc tccctgtgaa aaggaagcta 11160
   gggttctatg aatggacttc aaggttaaga agtcacataa atcccacagg cactgttttg 11220
   cttcagctag aaaatacaat gcagatgtca ttaaaagact tactttaaaa tgtttatttt 11280
   attgccaact actacttcct gtccaccttt ttctccattc actttaaaag ctcaaggcta 11340
   ggtggctcat gcctgtaatc ccagcacttt gggaggctga ggcgggcaga tcacctgagg 11400
   tcgggacttt gagacccgcc tggacaacat ggtgaaaccc catttctaat aaaaatataa 11460
   aaattagcca ggtgtggtgg cgcacctgtg gtcccagcta ctctgggggc tgaggcatga 11520
   gaatcgcttg aacccgggag tggaggttgc attgagctga gatcatgcca cctcactcca 11580
   gcctgggcaa caaagattcc atctcaaaaa aaaaaaaaaa gccaggcaca gtggctcatg 11640
   cctggaatcc cagcactttt ggaagctgag gcaggcagat cacttgaggt taggatttca 11700
   agaccagcct ggctaacata gtaaagccct gtctctacta aaaatacaaa aattagccag 11760
   gtatggtggc gagcttctgt agccccagct actcaggaga ctgaggcagg agaatcactt 11820
   gaacccggga agtggggggg tgcagtgacc caagatcacg ccactgcatt ccagcctggg 11880
   caacagagca agactccatc tcaaaaaaaa aagttctatt tccttgaata aaattttccg 11940
   aagtttaaac tttaggaata aaactattaa acccgtattt actcatccag atacccaccc 12000
   cccttgttga gattctctcc caattatcaa aatgtgtagc atatttaact accaagagct 12060
   aaacatcatt aagactgaaa tgtattaaga aggatgtata ggccaggcac ggtgtctcac 12120
   gcctgtaatc ccaacacttt gggaggccaa gtcgggcgga tcacgaggtc aggagatgga 12180
   gaccatcctg gccaacatgg tgaaaccccc tctctactaa aaatacaaaa attagccagg 12240
   caggtggcag gcacctgtaa tcccagctac tccagaggct gaggcaggac aatcacttga 12300
   acctgggagg cagaggctgc agtgagctga ggttgtacca attgcactcc agcctaggta 12360
   acgagcaaca ctccatctca aaaaaagaaa aaaaaaaaga tgtataattt ggaactgtta 12420
   agaggcattt taaaga 12436

<210> 2
<211> 429
<212> PRT

<213>  Homo sapiens

<400>  2

<210>  3
<211>  1290
<212>  DNA
<213>  Homo sapiens

<400>  3
atgcagctga ggaatcccga gctccacctg ggctgtgctc tggctctgcg gttcctggcc      60

ctcgtgtcct gggacatccc tggcgctagg gccctcgata acggactggc ccggaccccc     120

acaatgggat ggctccactg ggaaaggttc atgtgcaatc tggactgtca ggaggaaccc     180

gactcctgca tcagcgaaaa gctcttcatg gagatggccg agctgatggt gagcgagggc     240

tggaaggacg ccggctacga gtatctgtgc atcgatgact gctggatggc ccctcaaagg 300

gactccgaag gcaggctgca ggctgatccc caaaggtttc cccacggaat ccggcagctc 360

gccaactacg tgcattccaa gggcctcaag ctcggcatct acgccgacgt gggcaacaaa 420

acatgcgccg gattccccgg cagcttcggc tactacgaca tcgacgccca gacattcgct 480

gattggggag tggacctgct gaagttcgac ggctgttact gcgattccct ggaaaacctg 540

gccgacggct acaaacacat gtccctcgcc ctgaaccgga caggcaggtc catcgtgtac 600

agctgcgagt ggcccctgta catgtggcct ttccagaagc ccaactacac agagatcagg 660

cagtactgca accactggag gaacttcgct gacatcgacg actcctggaa gagcatcaag 720

agcatcctgg actggaccag cttcaaccag gagaggatcg tggacgtggc tggacccgga 780

ggctggaacg accccgatat gctggtgatt ggcaacttcg gactgagctg gaaccagcag 840

gtgacccaga tggccctgtg ggccattatg gccgctcccc tgttcatgtc caacgacctg 900

aggcacatca gcccccaggc caaggctctg ctgcaggaca aggatgtgat cgccatcaac 960

caggaccccc tgggcaagca gggctaccag ctgaggcaag gagataactt cgaggtgtgg 1020

gagaggcccc tgtccggact ggcttgggcc gtggccatga tcaatcggca ggagatcggc 1080

ggaccccggt cctacaccat tgctgtggcc agcctgggaa aaggagtcgc ctgcaacccc 1140

gcctgcttca ttacccagct gctccccgtg aagcggaagc tgggcttcta tgagtggacc 1200

agcaggctga ggtcccatat caatcctacc ggcaccgtcc tcctccagct cgagaatacc 1260

atgcagatga gcctcaagga tctgctgtga 1290

## Description

### TECHNICAL FIELD

Principles and embodiments of the present invention relate generally to the use of pharmacological chaperones for the treatment of lysosomal storage disorders, particularly the use of migalastat for the treatment of Fabry disease.

### REFERENCE TO THE SEQUENCE LISTING

The Sequence Listing text file submitted herewith, identified as "00790758.TXT" (22 Kb, created February 1, 2019), is hereby incorporated by reference.

### BACKGROUND

Fabry disease is a progressive, X-linked inborn error of glycosphingolipid metabolism caused by a deficiency in the lysosomal enzyme α-galactosidase A (a-Gal A) as a result of mutations in the α-Gal A gene (*GLA*). Despite being an X-linked disorder, females can express varying degrees of clinical manifestations. Fabry is a rare disease with incidence estimated between 1 in 40,000 males to 1 in 117,000 in the general population. Moreover, there are variants of later onset phenotype of Fabry disease that can be under-diagnosed, as they do not present with classical signs and symptoms. This, and newborn screening for Fabry disease, suggests that the actual incidence of Fabry disease can be higher than currently estimated.

Untreated, life expectancy in Fabry patients is reduced and death usually occurs in the fourth or fifth decade because of vascular disease affecting the kidneys, heart and/or central nervous system. The enzyme deficiency leads to intracellular accumulation of the substrate, globotriaosylceramide (GL-3) in the vascular endothelium and visceral tissues throughout the body. Gradual deterioration of renal function and the development of azotemia, due to glycosphingolipid deposition, usually occur in the third to fifth decades of life, but can occur as early as in the second decade. Renal lesions are found in both hemizygous (male) and heterozygous (female) patients.

Cardiac disease as a result of Fabry disease occurs in most males and many females. Early cardiac findings include left ventricular enlargement, valvular involvement and conduction abnormalities. Mitral insufficiency is the most frequent valvular lesion typically present in childhood or adolescence. Cerebrovascular manifestations result primarily from multifocal small-vessel involvement and can include thromboses, transient ischemic attacks, basilar artery ischemia and aneurysm, seizures, hemiplegia, hemianesthesia, aphasia, labyrinthine disorders, or cerebral hemorrhages. Average age of onset of cerebrovascular manifestations is 33.8 years. Personality change and psychotic behavior can manifest with increasing age.

The current approved treatment for Fabry disease is enzyme replacement therapy ("ERT"). Two α-Gal A products are currently available for the treatment of Fabry disease: agalsidase alfa (Replagal^{®}, Shire Human Genetic Therapies) and agalsidase beta (Fabrazyme^{®}; Sanofi Genzyme Corporation). These two forms of ERT are intended to compensate for a patient's inadequate α-Gal A activity with a recombinant form of the enzyme, administered intravenously. While ERT is effective in many settings, the treatment also has limitations. For example, these two α-Gal A products have not been demonstrated to decrease sufficient risk of stroke, cardiac muscle responds to treatment slowly, and GL-3 elimination from some of the cell types of the kidneys is limited.

Moreover, patients with the classic Fabry phenotype tend to have lower baseline α-Gal A activity, multiorgan system involvement and more severe disease manifestations at baseline.

Accordingly, there remains a need for therapies for the treatment of Fabry disease, particularly patients with classic Fabry disease.

### SUMMARY

Various aspects of the present invention relate to the treatment of patients having classic Fabry disease using migalastat. Such treatment can include reducing kidney GL-3, stabilizing renal function, reducing left ventricular mass (LVM), reducing plasma globotriaosylsphingosine (lyso-Gb₃) and/or treating gastrointestinal symptoms.

One aspect of the present invention pertains to a method of reducing kidney GL-3 in a patient having classic Fabry disease, the method comprising administering to the patient an effective amount of migalastat or salt thereof at a frequency of once every other day for reducing the patient's kidney GL-3. In one or more embodiments, the effective amount is about 100 mg to about 150 mg free base equivalent (FBE).

In one or more embodiments, the patient has elevated kidney interstitial capillary GL-3 prior to initiating administration of the migalastat or salt thereof.

In one or more embodiments, reducing kidney GL-3 comprises reducing GL-3 inclusions per kidney interstitial capillary.

In one or more embodiments, the patient has a mutation in α-Gal A selected from the group consisting of p.Ile253Th (I253T), p.Pro259Arg (P259R), p.Gly183Asp (G183D), p.Leu243Phe (L243F), p.Cys174Arg (C174R), p.Asp55Val/Gln57Leu (D55V/Q57L), p.Gly144Val (G144V), p.Arg328Gln (R301Q), p.Gly373Ser (G373S), p.Asp322Glu (D322E), p.Gly325Arg G325R and p.Tyr216Cys (Y216C).

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the patient is an ERT-naive patient.

In one or more embodiments, administration of the migalastat or salt thereof to a group of classic Fabry patients provides an average decrease in GL-3 inclusions per kidney interstitial capillary of at least about 0.5 after 6 months of the administration of the migalastat or salt thereof.

Another aspect of the present invention pertains to a method of treating classic Fabry disease in a patient in need thereof, the method comprising administering to the patient an effective amount of migalastat or salt thereof at a frequency of once every other day. In one or more embodiments, the effective amount is about 100 mg to about 150 mg FBE. In one or more embodiments, administering the migalastat or salt thereof reduces kidney GL-3 in the patient.

In one or more embodiments, the patient has elevated kidney interstitial capillary GL-3 prior to initiating administration of the migalastat or salt thereof.

In one or more embodiments, reducing kidney GL-3 comprises reducing GL-3 inclusions per kidney interstitial capillary.

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the patient is an ERT-naive patient.

In one or more embodiments, administration of the migalastat or salt thereof to a group of classic Fabry patients of provides an average decrease in GL-3 inclusions per kidney interstitial capillary of at least about 0.5 after 6 months of the administration of the migalastat or salt thereof.

Another aspect of the present invention pertains to a method of stabilizing renal function in a patient having classic Fabry disease, the method comprising administering to the patient an effective amount of migalastat or salt thereof at a frequency of once every other day for stabilizing the patient's renal function. In one or more embodiments, the effective amount is about 100 mg to about 150 mg FBE.

In one or more embodiments, the patient has renal impairment prior to initiating administration of the migalastat or salt thereof.

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naïve patient.

In one or more embodiments, administration of the migalastat or salt thereof to a group of classic Fabry patients provides a mean annualized rate of change in eGFR_{CKD-EPI} of greater than -1.0 mL/min/1.73 m² after 24 months of the administration of the migalastat or salt thereof.

Another aspect of the present invention pertains to a method of treating classic Fabry disease in a patient in need thereof, the method comprising administering to the patient an effective amount of migalastat or salt thereof at a frequency of once every other day. In one or more embodiments, the effective amount is about 100 mg to about 150 mg FBE. In one or more embodiments, administering the migalastat or salt thereof stabilizes renal function in the patient.

In one or more embodiments, the patient has renal impairment prior to initiating administration of the migalastat or salt thereof.

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naive patient.

In one or more embodiments, administration of the migalastat or salt thereof to a group of classic Fabry patients provides a mean annualized rate of change in eGFR_{CKD-EPI} of greater than -1.0 mL/min/1.73 m² after 24 months of the administration of the migalastat or salt thereof.

Another aspect of the present invention pertains to a method of reducing LVM in a patient having classic Fabry disease, the method comprising administering to the patient an effective amount of migalastat or salt thereof at a frequency of once every other day for reducing the patient's LVM. In one or more embodiments, the effective amount is about 100 mg to about 150 mg FBE.

In one or more embodiments, the patient has left ventricular hypertrophy (LVH) prior to initiating administration of the migalastat or salt thereof.

In one or more embodiments, reducing LVM comprises reducing left ventricular mass index (LVMi).

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naive patient.

In one or more embodiments, administration of the migalastat or salt thereof to a group of classic Fabry patients provides an average decrease in LVMi of at least about 5 g/m² after 24 months of the administration of the migalastat or salt thereof.

Another aspect of the present invention pertains to a method of treating classic Fabry disease in a patient in need thereof, the method comprising administering to the patient an effective amount of migalastat or salt thereof at a frequency of once every other day. In one or more embodiments, the effective amount is about 100 mg to about 150 mg FBE. In one or more embodiments, administering the migalastat or salt thereof reduces the patient's LVM.

In one or more embodiments, the patient has LVH prior to initiating administration of the migalastat or salt thereof.

In one or more embodiments, reducing LVM comprises reducing LVMi.

In one or more embodiments, the patient has a mutation in α-Gal A selected from the group consisting of I253T, P259R, G183D, L243F, C174R, DSSV/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naive patient.

In one or more embodiments, administration of the migalastat or salt thereof to a group of classic Fabry patients provides an average decrease in LVMi of at least about 5 g/m² after 24 months of the administration of the migalastat or salt thereof.

Another aspect of the present invention pertains to a method of reducing plasma lyso-Gb₃ in a patient having classic Fabry disease, the method comprising administering to the patient an effective amount of migalastat or salt thereof at a frequency of once every other day for reducing the patient's plasma lyso-Gb₃. In one or more embodiments, the effective amount is about 100 mg to about 150 mg FBE.

In one or more embodiments, the patient has elevated plasma lyso-Gb₃ prior to initiating administration of the migalastat or salt thereof.

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naive patient.

In one or more embodiments, wherein administration of the migalastat or salt thereof to a group of classic Fabry patients provides an average decrease in plasma lyso-Gb₃ of at least about 15 nmol/L after 24 months of the administration of the migalastat or salt thereof.

Another aspect of the present invention pertains to a method of treating classic Fabry disease in a patient in need thereof, the method comprising administering to the patient an effective amount of migalastat or salt thereof at a frequency of once every other day. In one or more embodiments, the effective amount is about 100 mg to about 150 mg FBE. In one or more embodiments, administering the migalastat or salt thereof reduces plasma lyso-Gb₃ in the patient.

In one or more embodiments, the patient has elevated plasma lyso-Gb₃ prior to initiating administration of the migalastat or salt thereof.

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naive patient.

In one or more embodiments, wherein administration of the migalastat or salt thereof to a group of classic Fabry patients provides an average decrease in plasma lyso-Gb₃ of at least about 15 nmol/L after 24 months of the administration of the migalastat or salt thereof.

Another aspect of the present invention pertains to a method of treating gastrointestinal symptoms in a patient having classic Fabry disease, the method comprising administering to the patient a formulation comprising an effective amount of migalastat or salt thereof at a frequency of once every other day for treating the gastrointestinal symptoms. In one or more embodiments, the effective amount is about 100 mg to about 150 mg FBE.

In one or more embodiments, the patient has diarrhea prior to initiating administration of the migalastat or salt thereof.

In one or more embodiments, treating one or more gastrointestinal symptoms in the patient comprises reducing symptoms of diarrhea.

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naïve patient.

In one or more embodiments, administration of the migalastat or salt thereof to a group of classic Fabry patients provides an average decrease in Gastrointestinal Symptoms Rating Scale for Dianhea (GSRS-D) of at least about 0.5 after 24 months of the administration of the migalastat or salt thereof.

Another aspect of the present invention pertains to a method of treating classic Fabry disease in a patient in need thereof, the method comprising administering to the patient an effective amount of migalastat or salt thereof at a frequency of once every other day. In one or more embodiments, the effective amount is about 100 mg to about 150 mg FBE. In one or more embodiments, administering the migalastat or salt thereof treats one or more gastrointestinal symptoms in the patient.

In one or more embodiments, the patient has diarrhea prior to initiating administration of the migalastat or salt thereof.

In one or more embodiments, treating one or more gastrointestinal symptoms in the patient comprises reducing symptoms of diarrhea.

In one or more embodiments, the patient has a mutation in α-Gal A selected from the group consisting of I253T, P259R, G183D, L243F, C174R, D55V/Q57L, G144V, R301Q, G373S, D322E, G325R and Y216C.

In one or more embodiments, the migalastat or salt thereof enhances α-Gal A activity.

In one or more embodiments, the effective amount is about 123 mg FBE.

In one or more embodiments, the effective amount is about 123 mg of migalastat free base.

In one or more embodiments, the salt of migalastat is migalastat hydrochloride.

In one or more embodiments, the effective amount is about 150 mg of migalastat hydrochloride.

In one or more embodiments, the migalastat or salt thereof is in an oral dosage form. In one or more embodiments, the oral dosage form comprises a tablet, a capsule or a solution.

In one or more embodiments, the migalastat or salt thereof is administered for at least 6 months.

In one or more embodiments, the migalastat or salt thereof is administered for at least 24 months.

In one or more embodiments, the patient is an ERT-naive patient.

In one or more embodiments, administration of the migalastat or salt thereof to a group of classic Fabry patients provides an average decrease in GSRS-D of at least about 0.5 after 24 months of the administration of the migalastat or salt thereof.

### BRIEF DESCRIPTION OF THE DRAWINGS

Further features of the present invention will become apparent from the following written description and the accompanying figures, in which:
FIGS. 1A-1E show the full DNA sequence of the human wild-type *GLA* gene (SEQ ID NO: 1);
FIG. 2 shows the wild-type α-Gal A protein (SEQ ID NO: 2);
FIG. 3 shows the nucleic acid sequence encoding the wild-type α-Gal A protein (SEQ ID NO: 3); and

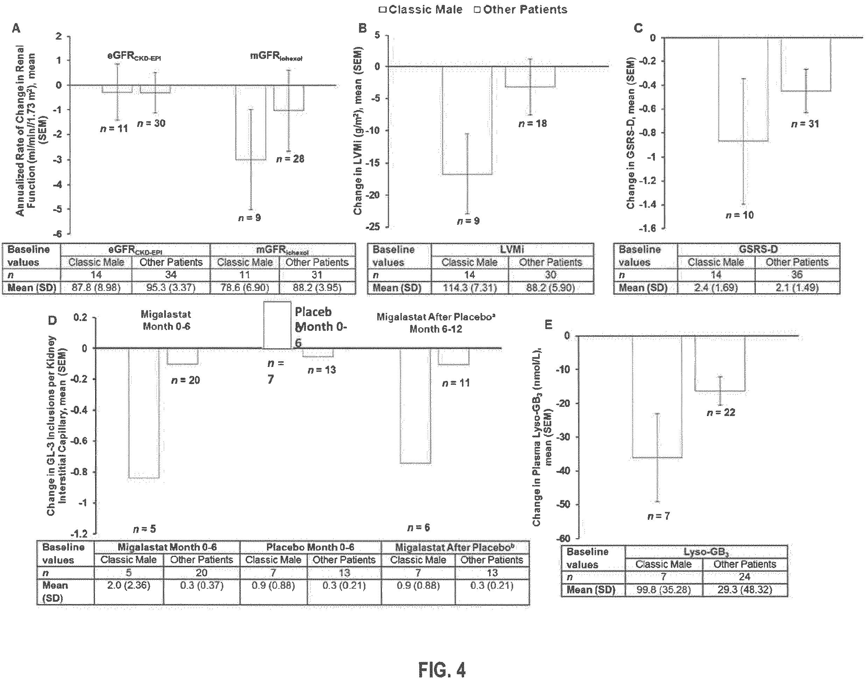

FIG4. 4A-4E show measurements of disease severity in classic male and other patient (non-classic males and females) subgroups. (A) Mean annualized rate of change in GFR from baseline/month 6 to month 24. (B) Mean change in LVMi from baseline/month 6 to month 24. (C) Mean change in GSRS-D from baseline/month 6 to month 24. (D) Mean change from baseline to month 12 in average number of GL-3 inclusion per interstitial capillary. Within each subgroup (classic males and other), patients are grouped according to treatment allocation (migalastat to migalastat or placebo to migalastat). (E) Mean change from baseline/month 6 to month 24 in plasma lyso-Gb₃. In FIGS. 4A-4E, ^{a}Group is comprised of patients who switched from placebo to migalastat at month 6 and ^{b}Data are mean (SD) number of GL-3 inclusions per interstitial capillary at month 6 (*i.e*., after 6 months of placebo treatment.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

Various aspects of the present invention pertain to dosing regimens for the administration of pharmacological chaperones such as migalastat for the treatment of classic Fabry disease. In one or more embodiments, the dosing regimens of migalastat reduce kidney GL-3, stabilize renal function, reduce LVM, reduce plasma lyso-Gb₃ and/or treat gastrointestinal symptoms.

### Definitions

The terms used in this specification generally have their ordinary meanings in the art, within the context of this invention and in the specific context where each term is used. Certain terms are discussed below, or elsewhere in the specification, to provide additional guidance to the practitioner in describing the compositions and methods of the invention and how to make and use them.

The term "Fabry disease" refers to an X-linked inborn error of glycosphingolipid catabolism due to deficient lysosomal α-Gal A activity. This defect causes accumulation of the substrate globotriaosylceramide ("GL-3", also known as Gb3 or ceramide trihexoside) and related glycosphingolipids in vascular endothelial lysosomes of the heart, kidneys, skin, and other tissues. Another substrate of the enzyme is plasma globotriaosylsphingosine ("plasma lyso-Gb3").

The term "classic Fabry disease" refers to patients with multiorgan system involvement. In one or more embodiments, the patient also has a residual peripheral blood mononuclear cell (PBMC) α-Gal A activity <3% of normal.

In various embodiments, multiple organ system involvement can be determined at baseline based on medical history. In other embodiments, multiple organ system involvement can be determined by identifying patients with cardiac, central nervous system, neurological pain and/or gastrointestinal systems at baseline, and then further identifying the patient as having renal symptoms due to a urine protein at baseline > 150 mg/24 hours or due to a baseline eGFR < 90 mL/min/1.73m².

A summary of exemplary criteria for identifying classic males in an ERT-naive trial and an ERT-experienced trial is shown in the chart below:

| | | ERT-Naïve | ERT-Experienced |
|---|---|---|---|
| 1 | Multi-organ disease at baseline based on medical history (renal, cardiac, CNS, neuro pain, and GI) | Multi-organ (≥ 2 organs) | Multi-organ (≥ 2 organs) |
| | But, if there medical history and if renal was not captured, then: | | |
| 2 | Urine protein at baseline | > 150 mg/24 hours | > 150 mg/24 hours |
| | Baseline eGFR | <90 mL/min/1.73m² | <90 mL/min/1.73m² |
| 3 | WBC α-gal-A at baseline | <3% WT | *NA |

| | | | |
|---|---|---|---|
| *Different criteria was used in the ERT-experienced trial because ERT use immediately prior to study entry would influence the WBC α-gal-A values at baseline. | | | |

Thus, in one set of exemplary criteria for the ERT-naïve trial, the classic patients had multi-organ involvement at baseline and WBC α-gal-A at baseline <3% of normal (i.e. 1 and 3 in the chart above). In another exemplary set of criteria for the ERT-naive trial, the classic patients had urine protein at baseline > 150 mg/24 hours or baseline eGFR <90 mL/min/1.73m², as well as WBC α-gal-A at baseline <3% of normal (i.e. 2 and 3 in the chart above).

In the exemplary criteria for the ERT-experienced trial, the classic patients had multi-organ involvement at baseline, or urine protein at baseline > 150 mg/24 hours, or baseline eGFR <90 mL/min/1.73m² (i.e. 1 or 2 in the chart above).

A "carrier" is a female who has one X chromosome with a defective *GLA* gene and one X chromosome with the normal gene and in whom X chromosome inactivation of the normal allele is present in one or more cell types. A carrier is often diagnosed with Fabry disease.

A "patient" refers to a subject who has been diagnosed with or is suspected of having a particular disease. The patient may be human or animal.

A "Fabry patient" refers to an individual who has been diagnosed with or suspected of having Fabry disease and has a mutated α-Gal A as defined further below. Characteristic markers of Fabry disease can occur in male hemizygotes and female carriers with the same prevalence, although females typically are less severely affected.

The term "ERT-naive patient" refers to a Fabry patient that has never received ERT or has not received ERT for at least 6 months prior to initiating migalastat therapy.

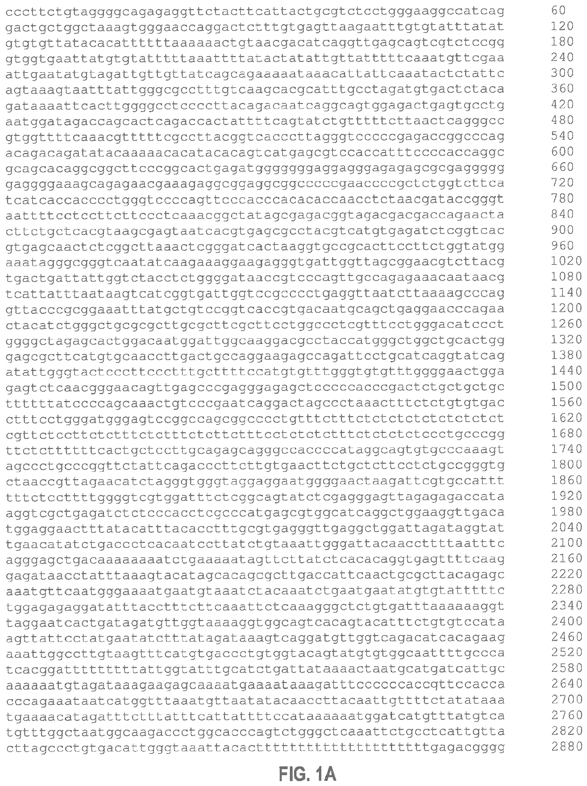

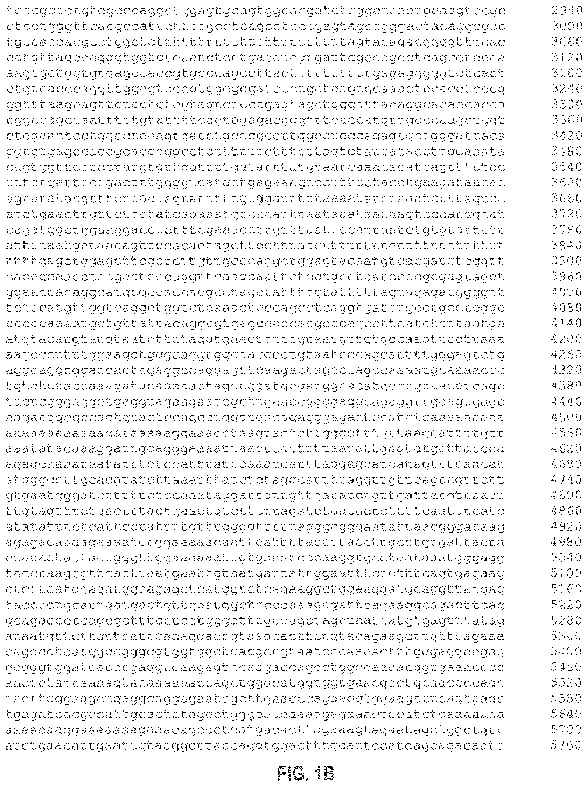

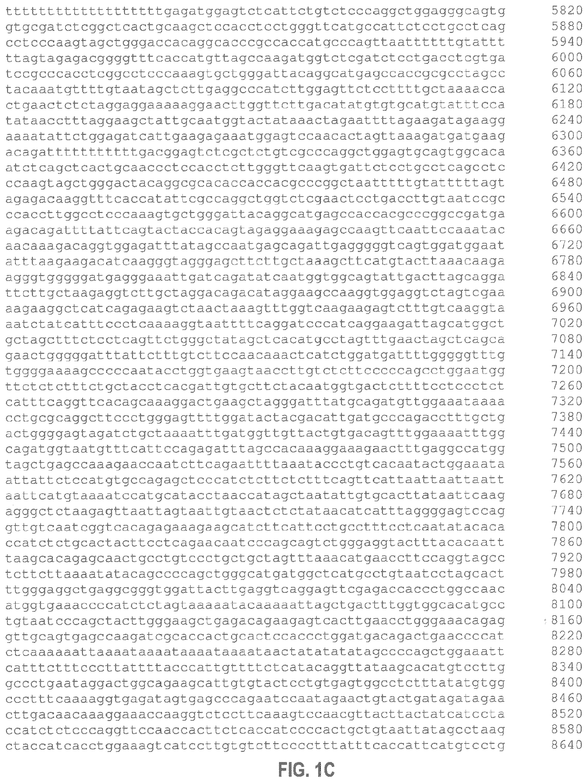

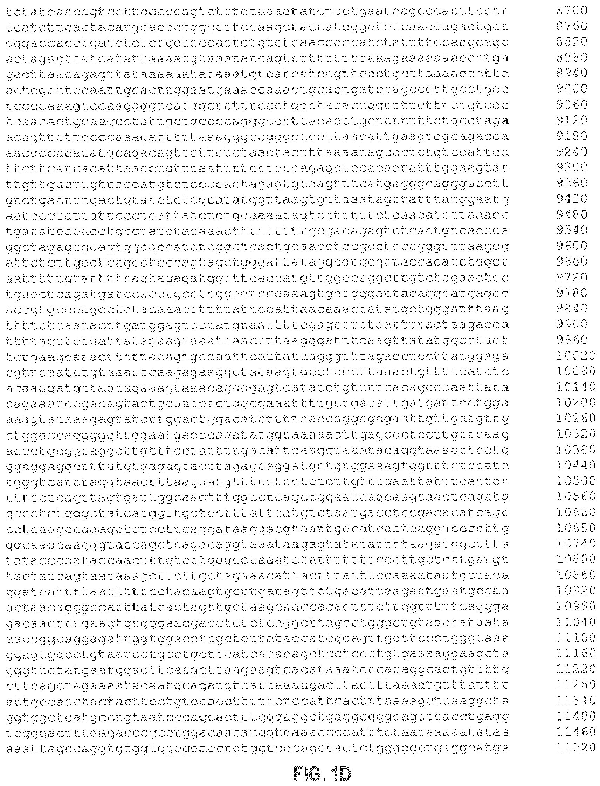

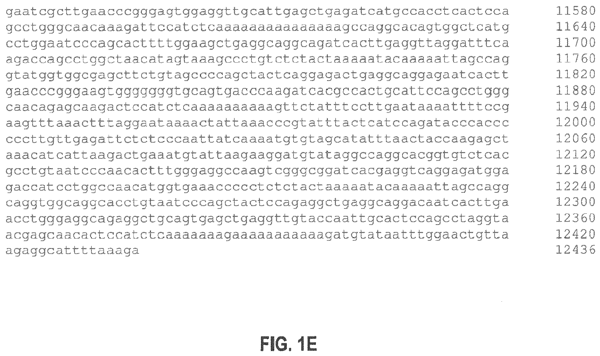

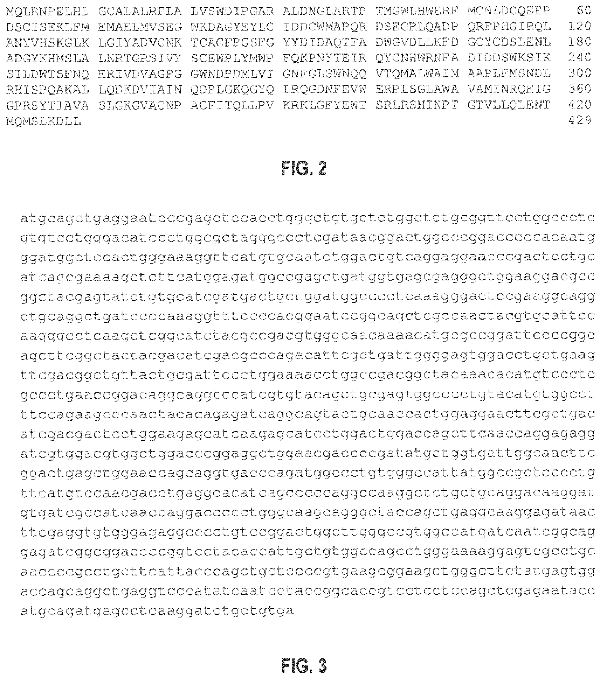

Human α-galactosidase A (a-Gal A) refers to an enzyme encoded by the human *GLA* gene. The full DNA sequence of α-Gal A, including introns and exons, is available in GenBank Accession No. X14448.1 and shown in SEQ ID NO: 1 and FIGS. 1A-1E. The human α-Gal A enzyme consists of 429 amino acids and is available in GenBank Accession Nos. X14448.1 and U78027.1 and shown in SEQ ID NO: 2 and FIG. 2. The nucleic acid sequence that only includes the coding regions (i.e. exons) of SEQ ID NO: 1 is shown in FIG. 3 (SEQ ID NO: 3).

The term "mutant protein" includes a protein which has a mutation in the gene encoding the protein which results in the inability of the protein to achieve a stable conformation under the conditions normally present in the endoplasmic reticulum. The failure to achieve a stable conformation results in a substantial amount of the enzyme being degraded, rather than being transported to the lysosome. Such a mutation is sometimes called a "conformational mutant." Such mutations include, but are not limited to, missense mutations, and in-frame small deletions and insertions.

As used herein in one embodiment, the term "mutant α-Gal A" includes an α-Gal A which has a mutation in the gene encoding α-Gal A which results in the inability of the enzyme to achieve a stable conformation under the conditions normally present in the endoplasmic reticulum. The failure to achieve a stable conformation results in a substantial amount of the enzyme being degraded, rather than being transported to the lysosome.

As used herein, the term "pharmacological chaperone" ("PC") refers to any molecule including a small molecule, protein, peptide, nucleic acid, carbohydrate, etc. that specifically binds to a protein and has one or more of the following effects: (i) enhances the formation of a stable molecular conformation of the protein; (ii) induces trafficking of the protein from the endoplasmic reticulum to another cellular location, preferably a native cellular location, *i.e.,* prevents endoplasmic reticulum-associated degradation of the protein; (iii) prevents aggregation of misfolded proteins; and/or (iv) restores or enhances at least partial wild-type function and/or activity to the protein. A compound that specifically binds to *e.g.,* α-Gal A, means that it binds to and exerts a chaperone effect on the enzyme and not a generic group of related or unrelated enzymes. More specifically, this term does not refer to endogenous chaperones, such as BiP, or to non-specific agents which have demonstrated non-specific chaperone activity against various proteins, such as glycerol, DMSO or deuterated water, *i.e.,* chemical chaperones. In one or more embodiments of the present invention, the PC may be a reversible competitive inhibitor.

A "competitive inhibitor" of an enzyme can refer to a compound which structurally resembles the chemical structure and molecular geometry of the enzyme substrate to bind the enzyme in approximately the same location as the substrate. Thus, the inhibitor competes for the same active site as the substrate molecule, thus increasing the Km. Competitive inhibition is usually reversible if sufficient substrate molecules are available to displace the inhibitor, *i.e.,* competitive inhibitors can bind reversibly. Therefore, the amount of enzyme inhibition depends upon the inhibitor concentration, substrate concentration, and the relative affinities of the inhibitor and substrate for the active site.

As used herein, the term "specifically binds" refers to the interaction of a pharmacological chaperone with a protein such as α-Gal A, specifically, an interaction with amino acid residues of the protein that directly participate in contacting the pharmacological chaperone. A pharmacological chaperone specifically binds a target protein, *e.g.,* α-Gal A, to exert a chaperone effect on the protein and not a generic group of related or unrelated proteins. The amino acid residues of a protein that interact with any given pharmacological chaperone may or may not be within the protein's "active site." Specific binding can be evaluated through routine binding assays or through structural studies, *e.g.,* co-crystallization, NMR, and the like. The active site for α-Gal A is the substrate binding site.

"Deficient α-Gal A activity" refers to α-Gal A activity in cells from a patient which is below the normal range as compared (using the same methods) to the activity in normal individuals not having or suspected of having Fabry or any other disease (especially a blood disease).

As used herein, the terms "enhance α-Gal A activity" or "increase α-Gal A activity" refer to increasing the amount of α-Gal A that adopts a stable conformation in a cell contacted with a pharmacological chaperone specific for the α-Gal A, relative to the amount in a cell (preferably of the same cell-type or the same cell, *e.g.,* at an earlier time) not contacted with the pharmacological chaperone specific for the α-Gal A. This term also refers to increasing the trafficking of α-Gal A to the lysosome in a cell contacted with a pharmacological chaperone specific for the α-Gal A, relative to the trafficking of α-Gal A not contacted with the pharmacological chaperone specific for the protein. These terms refer to both wild-type and mutant α-Gal A. In one embodiment, the increase in the amount of α-Gal A in the cell is measured by measuring the hydrolysis of an artificial substrate in lysates from cells that have been treated with the PC. An increase in hydrolysis is indicative of increased α-Gal A activity.

The term "a-Gal A activity" refers to the normal physiological function of a wild-type α-Gal A in a cell. For example, α-Gal A activity includes hydrolysis of GL-3.

A "responder" is an individual diagnosed with or suspected of having a lysosomal storage disorder, such, for example Fabry disease, whose cells exhibit sufficiently increased α-Gal A activity, respectively, and/or amelioration of symptoms or enhancement in surrogate markers, in response to contact with a PC. Non-limiting examples of enhancements in surrogate markers for Fabry are lyso-Gb3 and those disclosed in U.S. Patent Application Publication No. US 2010/0113517.

Non-limiting examples of enhancements in surrogate markers for Fabry disease disclosed in U.S. 2010/0113517 include increases in α-Gal A levels or activity in cells (*e.g.,* fibroblasts) and tissue; reductions in of GL-3 accumulation; decreased plasma concentrations of homocysteine and vascular cell adhesion molecule-1 (VCAM-1); decreased GL-3 accumulation within myocardial cells and valvular fibrocytes; reduction in plasma lyso-Gb3; reduction in cardiac hypertrophy (especially of the left ventricle), amelioration of valvular insufficiency, and arrhythmias; amelioration of proteinuria; decreased urinary concentrations of lipids such as CTH, lactosylceramide, ceramide, and increased urinary concentrations of glucosylceramide and sphingomyelin; the absence of laminated inclusion bodies (Zebra bodies) in glomerular epithelial cells; enhancements in renal function; mitigation of hypohidrosis; the absence of angiokeratomas; and enhancements in hearing abnormalities such as high frequency sensorineural hearing loss progressive hearing loss, sudden deafness, or tinnitus. Enhancements in neurological symptoms include prevention of transient ischemic attack (TIA) or stroke; and amelioration of neuropathic pain manifesting itself as acroparaesthesia (burning or tingling in extremities). Another type of clinical marker that can be assessed for Fabry disease is the prevalence of deleterious cardiovascular manifestations.

"Elevated kidney interstitial capillary GL-3" refers to any detectable level of interstitial capillary GL-3 in the kidney. In a kidney of a healthy person, no interstitial capillary GL-3 is accumulated. Conversely, due to a reduction in renal function, the level of interstitial capillary GL-3 in a kidney of, for example, a Fabry patient is elevated - detectable by using pathology in kidney biopsies.

As used herein, the phrase "stabilizing renal function" and similar terms refer to reducing decline in renal function and/or restoring renal function. As untreated Fabry patients are expected to have significant decreases in renal function, improvements in the rate of renal function deterioration and/or improvements in renal function demonstrate a benefit of migalastat therapy as described herein.

"Renal impairment" refers to a patient having an estimated glomerular filtration rate (eGFR) less than 90 mL/min/1.73m². Two of the most commonly used equations for calculating eGFR from serum creatinine are the Chronic Kidney Disease Epidemiology Collaboration (CKD-EPI) equation and the Modification of Diet in Renal Disease (MDRD), which are referred to as eGFR_{CKD-EPI} and eGFR_{MDRD}, respectively. The severity of chronic kidney disease has been defined in six stages:
a. (Stage 0) Normal kidney function - eGFR above 90 mL/min/1.73 m² and no proteinuria;
b. (Stage 1) - eGFR above 90 mL/min/1.73 m² with evidence of kidney damage;
   (Stage 2) (mild) - eGFR of 60 to 89 mL/min/1.73 m² with evidence of kidney damage;
c. (Stage 3) (moderate) - eGFR of 30 to 59 mL/min/1.73 m²;
d. (Stage 4) (severe) - eGFR of 15 to 29 mL/min/1.73 m²;
e. (Stage 5) kidney failure - eGFR less than 15 mL/min/1.73 m².

As used herein, the term "left ventricular hypertrophy" or "LVH" refers to a patient having an LVMi in the above-normal range. As the normal range of LVMi for a female is 43-95 g/m² and the normal range of LVMi for a male is 49-115 g/m², a female patient with LVH has an LVMi >95 g/m² and a male patient with LVH has an LVMi >115 g/m².

"Elevated plasma lyso-Gb₃" refers to plasma lyso-Gb₃ levels that are above the normal range. The normal range for plasma lyso-Gb₃ can vary depending on the particular assay used to assess the plasma lyso-Gb₃. In one or more embodiments, the normal range for plasma lyso-Gb₃ is 0.375 - 1.19 nmol/L and elevated plasma lyso-Gb₃ refers to levels of plasma lyso-Gb₃ greater than 1.19 nmol/L.

The phrase "pharmaceutically acceptable" refers to molecular entities and compositions that are physiologically tolerable and do not typically produce untoward reactions when administered to a human. In some embodiments, as used herein, the term "pharmaceutically acceptable" means ap0proved by a regulatory agency of the Federal or a state government or listed in the U.S. Pharmacopoeia or other generally recognized pharmacopoeia for use in animals, and more particularly in humans. The term "carrier" in reference to a pharmaceutical carrier refers to a diluent, adjuvant, excipient, or vehicle with which the compound is administered. Such pharmaceutical carriers can be sterile liquids, such as water and oils. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin, 18th Edition, or other editions.

The term "enzyme replacement therapy" or "ERT" refers to the introduction of a non-native, purified enzyme into an individual having a deficiency in such enzyme. The administered protein can be obtained from natural sources or by recombinant expression (as described in greater detail below). The term also refers to the introduction of a purified enzyme in an individual otherwise requiring or benefiting from administration of a purified enzyme, *e.g.,* suffering from enzyme insufficiency. The introduced enzyme may be a purified, recombinant enzyme produced in vitro, or protein purified from isolated tissue or fluid, such as, *e.g.,* placenta or animal milk, or from plants.

The term "ERT-naive patient" refers to a Fabry patient that has never received ERT or has not received ERT for at least 6 months prior to initiating migalastat therapy.

As used herein, the term "isolated" means that the referenced material is removed from the environment in which it is normally found. Thus, an isolated biological material can be free of cellular components, *i.e*., components of the cells in which the material is found or produced. In the case of nucleic acid molecules, an isolated nucleic acid includes a PCR product, an mRNA band on a gel, a cDNA, or a restriction fragment. In another embodiment, an isolated nucleic acid is preferably excised from the chromosome in which it may be found, and more preferably is no longer joined to non-regulatory, non-coding regions, or to other genes, located upstream or downstream of the gene contained by the isolated nucleic acid molecule when found in the chromosome. In yet another embodiment, the isolated nucleic acid lacks one or more introns. Isolated nucleic acids include sequences inserted into plasmids, cosmids, artificial chromosomes, and the like. Thus, in a specific embodiment, a recombinant nucleic acid is an isolated nucleic acid. An isolated protein may be associated with other proteins or nucleic acids, or both, with which it associates in the cell, or with cellular membranes if it is a membrane-associated protein. An isolated organelle, cell, or tissue is removed from the anatomical site in which it is found in an organism. An isolated material may be, but need not be, purified.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 10- or 5-fold, and more preferably within 2-fold of a given value. Numerical quantities given herein are approximate unless stated otherwise, meaning that the term "about" or "approximately" can be inferred when not expressly stated.

### Fabry Disease

Fabry disease is a rare, progressive and devastating X-linked lysosomal storage disorder. Mutations in the *GLA* gene result in a deficiency of the lysosomal enzyme α-Gal A, which is required for glycosphingolipid metabolism. Beginning early in life, the reduction in α-Gal A activity results in an accumulation of glycosphingolipids, including GL-3 and plasma lyso-Gb3, and leads to the symptoms and life-limiting sequelae of Fabry disease, including pain, gastrointestinal symptoms, renal failure, cardiomyopathy, cerebrovascular events, and early mortality. Early initiation of therapy and lifelong treatment provide an opportunity to slow disease progression and prolong life expectancy.

Fabry disease encompasses a spectrum of disease severity and age of onset, although it has traditionally been divided into 2 main phenotypes, "classic" and "late-onset". The classic phenotype has been ascribed primarily to males with undetectable to low α-Gal A activity and earlier onset of renal, cardiac and/or cerebrovascular manifestations. The late-onset phenotype has been ascribed primarily to males with higher residual α-Gal A activity and later onset of these disease manifestations. Heterozygous female carriers typically express the late-onset phenotype but depending on the pattern of X-chromosome inactivation may also display the classic phenotype.

More than 1000 Fabry disease-causing *GLA* mutations have been identified. Approximately 60% are missense mutations, resulting in single amino acid substitutions in the α-Gal A enzyme. Missense *GLA* mutations often result in the production of abnormally folded and unstable forms of α-Gal A. Normal cellular quality control mechanisms in the endoplasmic reticulum block the transit of these abnormal proteins to lysosomes and target them for premature degradation and elimination. Many missense mutant forms are targets for migalastat, an α-Gal A-specific pharmacological chaperone.

The clinical manifestations of Fabry disease span a broad spectrum of severity and roughly correlate with a patient's residual α-Gal A levels. Patients with the classic phenotype experience disease of various organs, including the kidneys, heart and brain, with disease symptoms first appearing in adolescence and typically progressing in severity until death in the fourth or fifth decade of life. A number of recent studies suggest that there are a large number of undiagnosed males and females that have a range of Fabry disease symptoms, such as impaired cardiac or renal function and strokes, that usually first appear in adulthood. Individuals with this type of Fabry disease, referred to as late-onset Fabry disease, tend to have higher residual α-Gal A levels than classic Fabry patients. Individuals with late-onset Fabry disease typically first experience disease symptoms in adulthood, and often have disease symptoms focused on a single organ, such as enlargement of the left ventricle or progressive kidney failure. In addition, late-onset Fabry disease may also present in the form of strokes of unknown cause.

Fabry patients have progressive kidney impairment, and untreated patients exhibit end-stage renal impairment by the fifth decade of life. Deficiency in α-Gal A activity leads to accumulation of GL-3 and related glycosphingolipids in many cell types including cells in the kidney. GL-3 accumulates in podocytes, epithelial cells and the tubular cells of the distal tubule and loop of Henle. Impairment in kidney function can manifest as proteinuria and reduced glomerular filtration rate.

Because Fabry disease is rare, involves multiple organs, has a wide age range of onset, and is heterogeneous, proper diagnosis is a challenge. Awareness is low among health care professionals and misdiagnoses are frequent. Diagnosis of Fabry disease is most often confirmed on the basis of decreased α-Gal A activity in plasma or peripheral leukocytes (WBCs) once a patient is symptomatic, coupled with mutational analysis. In females, diagnosis is even more challenging since the enzymatic identification of carrier females is less reliable due to random X-chromosomal inactivation in some cells of carriers. For example, some obligate carriers (daughters of classically affected males) have α-Gal A enzyme activities ranging from normal to very low activities. Since carriers can have normal α-Gal A enzyme activity in leukocytes, only the identification of an α-Gal A mutation by genetic testing provides precise carrier identification and/or diagnosis.

Mutant forms of α-Gal A are considered to be amenable to migalastat are defined as showing a relative increase (+10 µM migalastat) of ≥1.20-fold and an absolute increase (+ 10 µM migalastat) of ≥ 3.0% wild-type (WT) when the mutant form of α-Gal A is expressed in HEK-293 cells (referred to as the "HEK assay") according to Good Laboratory Practice (GLP)-validated *in vitro* assay (GLP HEK or Migalastat Amenability Assay). Such mutations are also referred to herein as "HEK assay amenable" mutations.

Previous screening methods have been provided that assess enzyme enhancement prior to the initiation of treatment. For example, an assay using HEK-293 cells has been utilized in clinical trials to predict whether a given mutation will be responsive to pharmacological chaperone (*e.g.*, migalastat) treatment. In this assay, cDNA constructs are created. The corresponding α-Gal A mutant forms are transiently expressed in HEK-293 cells. Cells are then incubated ± migalastat (17 nM to 1 mM) for 4 to 5 days. After, α-Gal A levels are measured in cell lysates using a synthetic fluorogenic substrate (4-MU-α-Gal) or by western blot. This has been done for known disease-causing missense or small in-frame insertion/deletion mutations. Mutations that have previously been identified as responsive to a PC (*e.g*., migalastat) using these methods are listed in U.S. Patent No. 8,592,362.

### Pharmacological Chaperones

The binding of small molecule inhibitors of enzymes associated with LSDs can increase the stability of both mutant enzyme and the corresponding wild-type enzyme (see U.S. Pat. Nos. 6,274,597; 6,583,158; 6,589,964; 6,599,919; 6,916,829, and 7,141,582 all incorporated herein by reference). In particular, administration of small molecule derivatives of glucose and galactose, which are specific, selective competitive inhibitors for several target lysosomal enzymes, effectively increased the stability of the enzymes in cells in vitro and, thus, increased trafficking of the enzymes to the lysosome. Thus, by increasing the amount of enzyme in the lysosome, hydrolysis of the enzyme substrates is expected to increase. The original theory behind this strategy was as follows: since the mutant enzyme protein is unstable in the endoplasmic reticulum (Ishii et al., Biochem. Biophys. Res. Comm. 1996; 220: 812-815), the enzyme protein is retarded in the normal transport pathway (endoplasmic reticulum→Golgi apparatus→endosomes→lysosome) and prematurely degraded. Therefore, a compound which binds to and increases the stability of a mutant enzyme, may serve as a "chaperone" for the enzyme and increase the amount that can exit the endoplasmic reticulum and move to the lysosomes. In addition, because the folding and trafficking of some wild-type proteins is incomplete, with up to 70% of some wild-type proteins being degraded in some instances prior to reaching their final cellular location, the chaperones can be used to stabilize wild-type enzymes and increase the amount of enzyme which can exit the endoplasmic reticulum and be trafficked to lysosomes.

In one or more embodiments, the pharmacological chaperone comprises migalastat or salt thereof. As used herein, the compound migalastat, also known as 1-deoxygalactonojirimycin (1-DGJ) or (2R, 3S, 4R, 5S0-2-(hydroxymethyl)piperidine-3,4,5-triol, is a compound having the following chemical formula:

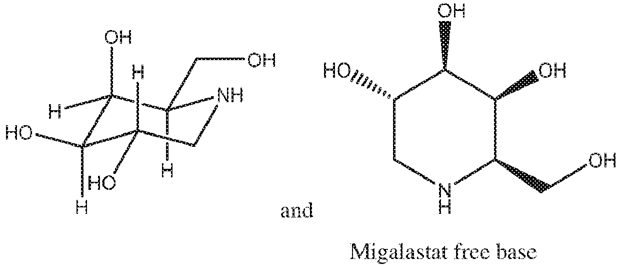

As discussed below, pharmaceutically acceptable salts of migalastat may also be used in the present invention. When a salt of migalastat is used, the dosage of the salt will be adjusted so that the dose of migalastat received by the patient is equivalent to the amount which would have been received had the migalastat free base been used. One example of a pharmaceutically acceptable salt of migalastat is migalastat HCl:

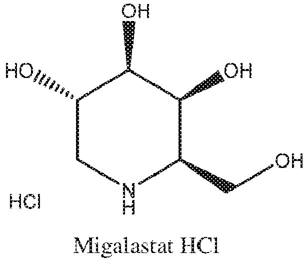

The term "migalastat" encompasses migalastat free base or a pharmaceutically acceptable salt thereof (e.g., migalastat HCl as shown above), unless specifically indicated to the contrary.

As used herein, the term "free base equivalent" or "FBE" refers to the amount of migalastat present in the migalastat or salt thereof. In other words, the term "FBE" means either an amount of migalastat free base, or the equivalent amount of migalastat free base that is provided by a salt of migalastat. For example, due to the weight of the hydrochloride salt, 150 mg of migalastat hydrochloride only provides as much migalastat as 123 mg of the free base form of migalastat. Other salts will have different conversion factors, depending on the molecular weight of the salt.

Migalastat is a low molecular weight iminosugar and is an analogue of the terminal galactose of GL-3. *In vitro* and *in vivo* pharmacologic studies have demonstrated that migalastat acts as a pharmacological chaperone, selectively and reversibly binding, with high affinity, to the active site of wild-type (WT) α-Gal A and specific mutant forms of α-Gal A, the genotypes of which are referred to as HEK assay amenable mutations. Migalastat binding stabilizes these mutant forms of α-Gal A in the endoplasmic reticulum facilitating their proper trafficking to lysosomes where dissociation of migalastat allows α-Gal A to reduce the level of GL-3 and other substrates. Approximately 35-50% of patients with Fabry disease have HEK assay amenable mutations; the majority of which are associated with the classic phenotype of the disease. A list of HEK assay amenable mutations includes at least those mutations listed in Table 1 below. In one or more embodiments, if a double mutation is present on the same chromosome (males and females), that patient is considered HEK assay amenable if the double mutation is present in one entry in Table 1 (*e.g*., D55V/Q57L). In some embodiments, if a double mutation is present on different chromosomes (only in females) that patient is considered HEK assay amenable if either one of the individual mutations is present in Table 1.

**Table 1**

| **Nucleotide change** | **Nucleotide change** | **Protein sequence change** |
|---|---|---|
| c.7C>G | c.C7G | L3V |
| c.8T>C | c.T8C | L3P |
| c.[11G>T; 620A>C] | c.G11T/A620C | R4M/Y207S |
| c.37G>A | c.G37A | A13T |
| c.37G>C | c.G37C | A13P |
| c.43G>A | c.G43A | A15T |
| c.44C>G | c.C44G | A15G |
| c.53T>G | c.T53G | F18C |
| c.58G>C | c.G58C | A2QP |
| c.59C>A | c.C59A | A20D |
| c.70T>C or c.70T>A | c.T70C or c.T70A | W24R |
| c.70T>G | c.T70G | W24G |
| c.72G>C or c.72G>T | c.G72C of c.G72T | W24C |
| c.95T>C | c.T95C | L32P |
| c.97G>T | c.G97T | D33Y |
| c98A>G | c.A98G | D33G |
| c.100A>G | c.A100G | N34D |
| c.101A>C | c.A101C | N34T |
| c.101A>G | c.A1010 | N34S |
| c.102T>G of c.102T>A | c.T102G or c.T102A | N34K |
| c.103G>C or c.103G>A | c.G103C or c.0103A | G35R. |
| c.104G>A | c.G104A | G35B |
| c.104G>T | c.G104T | G35V |
| c.107T>C | c.T107C | L36S |
| c.107T>G | c.T107G | L36W |
| c.108G>C or c.108G>T | c.G108C or c.G108T | L36F |
| c.109G>A | c.G109A | A37T |
| c.110C>T | c.C110T | A37V |
| c.122C>T | c.C122T | T41I |
| c.124A>C of c.124A>T | c.A124C or cA124T | M42L |
| c.124A>G | c.A124G | M42V |
| c.125T>A | c.T125A | M42K |
| c.125T>C | c.T125C | M42T |
| c.125T>G | c.T125G | M42R |
| c.126G>A or c.126G>C or c.126G>T | c.G126A or c.G126C or c.G126T | M42I |
| c.137A>C | c.A137C | H46P |
| c.142G>C | c.G142C | E48Q |
| c.152T>A | c.T152A | M31K |
| c.153G>A of c.153G>T or c.153G>C | c.G153A or c.G153T or c.G153C | M51I |
| c.157A>G | c.A157G | N53D |
| c.[157A>C; 158A>T] | c.A157C/A158T | N33L |
| c.160C>T | c.C160T | L54F |
| c.161T>C | c.T161C | L54P |
| c.164A>G | c.A164G | D55G |
| c.164A>T | c.A164T | D55V |
| c.[164A>T; 170A>T] | c.A164T/A170T | D55V/Q57L |
| c.167G>T | c.G167T | C56F |
| c.167G>A | c.G167A | C56Y |
| c.170A>T | c.A170T | Q57L |
| c.175G>A | c.G175A | E59K |
| c.178C>A | c.C178A | P60T |
| c.178C>T | c.C178T | P60S |
| c.179C>T | c.C179T | P60L |
| c.196G>A | c.G196A | B66K |
| c.197A>G | c.A197G | E66G |
| c.207C>A or c.207C>G | c.C207A or c.C207G | F69L |
| c.214A>G | c.A214G | M72V |
| c.216G>A or c.216G>T or c.216G>C | c.G216A or c.G216T or c.G216C | M72I |
| c.218C>T | c.C218T | A73V |
| c.227T>C | c.T227C | M76T |
| c.239G>A | c.G239A | G80D |
| c.247G>A | c.G247A | D83N |
| c.253G>A | c.G1253A | G85S |
| c.254G>A | c.G254A | G85D |
| c.[253G>A; 254G>A] | c.G253A/G254A | G85N |
| c.[253G>A; 254G>T; 255T>G] | c.G253A/G254T/T255G | G85M |
| c.261G>C of c.261G>T | c.G261C or c.G261T | B87D |
| c.265C>T | c.C265T | L89F |
| c.272T>C | c.T272C | I91T |
| c.288G>A or c.288G>T of c.288G>C | c.G288A or c.G288T or c.G288C | M96I |
| c.289G>C | c.G289C | A97P |
| c.290C>T | c.C290T | A97V |
| c305C>T | c.C305T | S102L |
| c311G>T | c.G311T | G104V |
| c.316C>T | c.C316T | L106F |
| c.322G>A | c.G322A | A108T |
| c.326A>G | c.A326G | D109G |
| c.334C>G | c.C334G | R1120 |
| c.333G>A | c.G335A | R112H |
| c.337T>A | c.T337A | F113I |
| c.337T>C of c.339T>A or c.339T>G | c.T337C or c.T339A or c.T339G | F113L |
| c.352C>T | c.C352T | R118C |
| c.361G>A | c.G361A | A121T |
| c.368A>G | c.A368G | Y123C |
| c.373C>T | c.C373T | H123Y |
| c374A>T | c.A374T | H125L |
| c.376A>G | c.A376G | S126G |
| c.383G>A | c.G383A | G128E |
| c.399T>G | c.T399G | I133M |
| c.404C>T | c.C404T | A135V |
| c.408T>A of c.408T>G | c.T408A or c.T408G | D136B |
| c.416A>G | c.A416G | N139S |
| c.419A>C | c.A419C | K140T |
| c.427G>A | c.G427A | A143T |
| c.431G>A | c.G431A | G144D |
| cA31G>T | c.G431T | G144V |
| c.434T>C | c.T434C | F145S |
| c.436C>T | c.C436T | P146S |
| c.437C>G | c.C437G | P146R |
| c.454T>C | c.T454C | Y152H |
| c.455A>G | c.A455G | Y152C |
| c.466G>A | c.G466A | A156T |
| c.467C>T | c.C467T | A156V |
| c.471G>C or c.471G>T | c.G471C or c.G471T | Q157H |
| cA84T>G | c.T484G | W162G |
| c.493G>C | c.G493C | D165H |
| c.494A>G | c.A494G | D1650 |
| c.[496C>G; 491T>G] | c.C496G/T4970 | L166G |
| c.496C>G | c.C496G | L166V |
| c.496_497delinsTC | c.496_497delinsTC | L166S |
| c.499C>G | c.C499G | L167V |
| c.506T>C | c.T506C | F169S |
| c.511G>A | c.G511A | G171S |
| c.520T>C | c.T520C | C174R |
| c.520T>G | c.T520G | C174G |
| c.525C>G or c.525C>A | c.C525G or c.C525A | D175B |
| c.539T>G | c.T539G | L180W |
| c.540G>C | c.G540C | L180F |
| c.548G>C | c.G548C | G183A |
| c.548G>A | c.G548A | G183D |
| c.550T>A | c.T550A | Y184N |
| c.551A>G | c.A551G | Y184C |
| c.553A>G | c.A553G | K185B |
| c.559A>G | c.A559G | M187V |
| c.559_564dup | c.559_564dup | p.M187_S188dup |
| c.560T>C | c.T560C | M187T |
| c.561G>T of c.561G>A of c.561G>C | c.G561T or c.G561A or c.G561C | M187I |
| c.572T>A | c.T572A | L191Q |
| c.581C>T | c.C581T | T194I |
| c.584G>T | c.G584T | G195V |
| c.586A>G | c.A586G | R196G |
| c.593T>C | c.T593C | I198T |
| c.595G>A | c.G595A | V199M |
| c.596T>C | c.T596C | V199A |
| c.596T>G | c.T396G | V199G |
| c.599A>G | c.A599G | Y200C |
| c.602C>T | c.C602T | S201F |
| c.602C>A | c.C602A | S201Y |
| c.608A>T | c.A608T | B203V |
| c.609G>C or c.609G>T | c.G609C or c.G609T | E203D |
| c.613C>A | c.C613A | P205T |
| c.613C>T | c.C613T | P205S |
| c.614C>T | c.C614T | P205L |
| c.619T>C | c.T619C | Y207H |
| c^20A>C | c.A620C | Y207S |
| c.623T>G | c.T623G | M208R |
| c.628C>T | c.C628T | P210S |
| c.629C>T | c.C629T | P210L |
| c.638A>G | c.A638G | K213R |
| c.638A>T | c.A638T | K213M |
| c.640C>T | c.C640T | P214S |
| c.641C>T | c.C641T | P214L |
| c.643A>G | cA643G | N215D |
| c.644A>G | c.A644G | N215S |
| c.644A>T | c.A644T | N215I |
| c.[644A>G; 937G>T] | c.A644G/G937T | N215S/D313Y |
| c.646T>G | c.T646G | Y216D |
| c.647A>G | c.A647G | Y216C |
| c.655A>C | c.A655C | 121%. |
| c.656T>A | c.T656A | I219N |
| c.656T>C | c.T656C | I219T |
| c.659G>A | c.G659A | R220Q |
| c.659G>C | c.G659C | R220P |
| c.662A>C | c.A662C | Q221P |
| c.671A>C | c.A671C | N224T |
| c.671A>G | c.A671G | N224S |
| c.673C>G | c.C673G | H225D |
| c.683A>G | c.A683G | N228S |
| c.687T>A or c.687T>G | c.TS87A or c.T687G | F229L |
| c.695T>C | c.T695C | I232T |
| c.713G>A | c.G713A | S238N |
| c.716T>C | c.T716C | I239T |
| c.720G>C of c.720G>T | c.G720C of c.G720T | K240N |
| c.724A>G | c.A724G | I242V |
| c.724A>T | c.A724T | I242F |
| c.725T>A | c.T725A | I242N |
| c.725T>C | c.T725C | I242T |
| c.728T>G | c.T728G | L243W |
| c.729G>C or c.729G>T | c.G729C of c.G729T | L243F |
| c.730G>A | c.G730A | D244N |
| c.730G>C | c.G730C | D244H |
| c.733T>G | c.T733G | W245G |
| c.740C>G | c.C740G | S247C |
| c.747C>G or c.747C>A | c.C747G or c.C747A | N249K |
| c.749A>C | c.A749C | Q2SOP |
| c.749A>G | c.A749G | Q250R |
| c.750G>C | c.G750C | Q250H |
| c.758T>C | c.T758C | I253T |
| c.758T>G | c.T758G | I253S |
| c.760-762delGTT | c.760_762delGTT | p.V254del |
| c.769G>C | c.G769C | A257P |
| c.770C>G | c.C770G | A237G |
| c.772G>C or c.772G>A | c.G772C of c.G772A | G258R |
| c.773G>T | c.G773T | G258V |
| c.776C>G | c.C776G | P259R |
| c.776C>T | c.C776T | P259L |
| c.779G>A | c.G779A | G260B |
| c.779G>C | c.G779C | G260A |
| c.781G>A | c.G781A | G261S |
| c.781G>C | c.G781C | G261R |
| c.781G>T | c.G781T | G261C |
| c.788A>G | c.A788G | N263S |
| c.790G>T | c.G790T | D264Y |
| c.794C>T | c.C794T | P265L |
| c.800T>C | c.T800C | M267T |
| c.805G>A | c.G805A | V269M |
| c.806T>C | c.T806C | V269A |
| c.809T>C | c.T809C | I270T |
| c.810T>G | c.T810G | I270M |
| c.811G>A | c.G811A | 02718 |
| c.[811G>A; 937G>T] | c.G811A/G937T | G271S/D313Y |
| c.812G>A | c.GB12A | G271D |
| c.823C>G | c.C823G | 1275V |
| c.827G>A | c.G827A | S276N |
| c.829T>G | c.T829G | W277G |
| c.831G>T or c.831G>C | c.G831T or c.G831C | W277C |
| c.832A>T | c.A832T | N278Y |
| c.835C>G | c.C835G | Q279B |
| c.838C>A | c.C838A | Q280K |
| c.840A>T of c.840A>C | c.A840T or c.A840C | Q280H |
| c.844A>G | c.A844G | T282A |
| c.845C>T | c.C845T | T282I |
| c.850A>G | c.A850G | M284V |
| c.851T>C | c.T851C | M284T |
| c.860G>T | c.G860T | W287L |
| c.862G>C | c.G862C | A288P |
| c.866T>G | c.T866G | I289S |
| c.868A>C of c.868A>T | c.A868C or c.A868T | M290L |
| c.869T>C | c.T869C | M290T |
| c.870G>A or c.870G>C or c.870G>T | c.G870A of c.G870C or c.G870T | M290I |
| c.871G>A | c.G871A | A291T |
| c.877C>A | c.C877A | P293T |
| c.881T>C | c.TB81C | L294S |
| c.884T>G | c.T884G | F295C |
| c.886A>G | c.A886G | M296V |
| c.886A>T of c.886A>C | c.A886T or c.A886C | M296L |
| c.88T>C | c.T887C | M296T |
| c.888G**>**A or c.888G>T or c.888G>C | c.G888A or c.G888T of c.G888C | M296I |
| c.893A>G | cA893G | N298S |
| c.897C>G or c.897C>A | c.C897G of c.C897A | D299B |
| c.898C>T | c.C898T | L300F |
| c.899T>C | c.T899C | L300P |
| c.901C>G | c.C901G | R301G |
| c.902G>C | c.G902C | R301P |
| c.902G>A | c.G902A | R301Q |
| c.902G>T | c.G902T | R301L |
| c.907A>T | c.A907T | I303F |
| c9O8T>A | c.T908A | I303N |
| c.911G>A | c.G911A | S304N |
| c.911G>C | c.G911C | S304T |
| c.919G>A | c.G919A | A307T |
| c.922A>G | cA922G | K308B |
| c.924A>T or c.924A>C | c.A924T or c.A924C | K308N |
| c525G>C | c.G925C | A309P |
| c.926C>T | c.C92CT | A3O9V |
| c.928C>T | c.C928T | L310F |
| c.931C>G | c.C931G | L311V |
| c.935A>G | c.A935G | Q312R |
| c.936G>T or c.936G>C | c.G936T or c.G936C | Q312H |
| c.937G>T | c.G937T | D313Y |
| c.[937G>T; 1232G>A] | c.G937T/G1232A | D313Y/G411D |
| c.938A>G | cA938G | D313G |
| c.946G>A | c.G946A | V316 |
| c547T>G | c.T947G | V316G |
| c.950T>C | c.T950C | I317T |
| c.955A>T | c.A955T | I319F |
| c.956T>C | c.T956C | I319T |
| c.939A>T | c.A959T | N3201 |
| c.962A>G | cA962G | Q321R |
| c.962A>T | c.A962T | Q321L |
| c.963G>C of c.963G>T | c.G963C or c.G963T | Q321H |
| c.964G>A | c.G964A | D322N |
| c.964G>C | c.G964C | D322H |
| c.966C>A or c.966C>G | c.C966A or c.C966G | D322B |
| c.968C>G | c.C968G | P323R |
| c.973G>A | c.G973A | G32SS |
| c.973G>C | c.G973C | G325R |
| c.978G>C of c.978G>T | c.G978C or c.G978T | K326N |
| c.979C>G | c.C979G | Q327E |
| c980A>T | c.A980T | Q327L |
| c.983G>C | c.G983C | G328A |
| c.989A>G | c.A989G | Q330R |
| c.1001G>A | c.G1001A | G334E |
| c.1010T>C | c.T1010C | F337S |
| c.1012G>A | c.G1012A | E338K |
| c.1016T>A | c.T1016A | V339E |
| c.1027C>A | c.C1027A | P343T |
| c.1028C>T | c.C1028T | P343L |
| c.1033T>C | c.T1033C | S345P |
| c.1046G>C | c.G1046C | W349S |
| c.1055C>G | c.C1055M | A352G |
| c.1055C>T | c.C1055T | A352V |
| c.1061T>A | c.T1061A | I354K |
| c.1066C>G | c.C1066G | R356G |
| c.1066C>T | c.C1066T | R356W |
| c.1067G>A | c.G1067A | R356Q |
| c.1067G>C | c.G1067C | R356P |
| c.1072G>C | c.G1072C | B358Q |
| c.1073A>C | c.A1073C | H338A |
| c.1073A>G | c.A1073G | E358G |
| c.1074G>T or c.1074G>C | c.C1074T or c.G1074C | E358D |
| c.1076T>C | c.T1076C | I359T |
| c.1078G>A | c.G1078A | G360S |
| c.1078G>T | c.G1078T | G360C |
| c.1079G>A | c.G1079A | G360D |
| c.1082G>A | c.G1082A | G361B |
| c.1082G>C | c.G1082C | G361A |
| c.1084C>A | c.C1084A | P362T |
| c.1085C>T | c.C1085T | P362L |
| c.1087C>T | c.C1087T | R363C |
| c.1088G>A | c.G1088A | R363H |
| c.1102G>A | c.G1102A | A368T |
| c.1117G>A | c.G1117A | G373S |
| c.1124G>A | c.G1124A | G375E |
| c.1153A>G | c.A1153G | T385A |
| c.1168G>A | c.G1168A | V390M |
| c.1172A>C | c.A1172C | K391T |
| c.1184G>A | c.G1184A | G395E |
| c.1184G>C | c.G1184C | G395A |
| c.1192G>A | c.G1192A | B398K |
| c.1202**_**1203insGACTTC | c.1202_1203insGACTTC | p.T400_S401dup |
| c.1208T>C | c.T1208C | L403S |
| c.1225C>G | c.C1225G | P409A |
| c.1225C>T | c.C1225T | P409S |
| c.1225C>A | c.C1225A | P409T |
| c.1228A>G | c.A1228G | T410A |
| c.1229C>T | c.C1229T | T410I |
| c.1232G>A | c.G1232A | G411D |
| c.1235C>A | c.C1235A | T412N |
| c.1253A>G | c.A1253G | E418G |
| c.1261A>G | c.A1261G | M421V |

### Dosing, Formulation and Administration

In one or more embodiments, the Fabry patient is administered migalastat or salt thereof at a frequency of once every other day (also referred to as "QOD"). In various embodiments, the doses described herein pertain to migalastat hydrochloride or an equivalent dose of migalastat or a salt thereof other than the hydrochloride salt In some embodiments, there doses pertain to the free base of migalastat. In alternate embodiments, these doses pertain to a salt of migalastat In further embodiments, the salt of migalastat is migalastat hydrochloride. The administration of migalastat or a salt of migalastat is referred to herein as "migalastat therapy".

The effective amount of migalastat or salt thereof can be in the range from about 100 mg FBE to about 150 mg FBE. Exemplary doses include about 100 mg FBE, about 105 mg FBE, about 110 mg FBE, about 115 mg FBE. about 120 mg FBE, about 123 mg FBE, about 125 mg FBE, about 130 mg FBE, about 135 mg FBE, about 140 mg FBE. about 145 mg FBE or about 150 mg FBE.

Again, it is noted that 150 mg of migalastat hydrochloride is equivalent to 123 mg of the free base form of migalastat Thus, in one or more embodiments, the dose is 150 mg of migalastat hydrochloride or an equivalent dose of migalastat or a salt thereof other than the hydrochloride salt administered at a frequency of once every other day. As set forth above, this dose is referred to as 123 mg FBE of migalastat In further embodiments, the dose is 150 mg of migalastat hydrochloride administered at a frequency of once every other day. In other embodiments, the dose is 123 mg of the migalastat free base administered at a frequency of once every other day.

In various embodiments, the effective amount is about 122 mg, about 128 mg, about 134 mg, about 140 mg, about 146 mg, about 150 mg, about 152 mg, about 159 mg, about 165 mg, about 171 mg, about 177 mg or about 183 mg of migalastat hydrochloride.

Accordingly, in various embodiments, migalastat therapy includes administering 123 mg FBE at a frequency of once every other day, such as 150 mg of migalastat hydrochloride every other day.

The administration of migalastat or salt thereof may be for a certain period of time. In one or more embodiments, the migalastat or salt thereof is administered for at least 28 days, such as at least 30, 60 or 90 days or at least 3, 4, 5, 6, 7, 8, 9, 10, 11, 12, 16, 20, 24, 30 or 36 months or at least 1, 2, 3, 4 or 5 years. In various embodiments, the migalastat therapy is long-term migalastat therapy of at least 6 months, such as at least 6, 7, 8, 9, 10, 11, 12, 16, 20, 24, 30 or 36 months or at least 1, 2, 3, 4 or 5 years.

Administration of migalastat or salt thereof according to the present invention may be in a formulation suitable for any route of administration, but is preferably administered in an oral dosage form such as a tablet, capsule or solution. As one example, the patient is orally administered capsules each containing 150 mg migalastat hydrochloride or an equivalent dose of migalastat or a salt thereof other than the hydrochloride salt.

In some embodiments, the PC (*e.g.,* migalastat or salt thereof) is administered orally. In one or more embodiments, the PC (*e.g.,* migalastat or salt thereof) is administered by injection. The PC may be accompanied by a pharmaceutically acceptable carrier, which may depend on the method of administration.

In one embodiment of the invention, the PC (*e.g.,* migalastat or salt thereof) is administered as monotherapy, and can be in a form suitable for any route of administration, including *e.g.,* orally in the form tablets or capsules or liquid, in sterile aqueous solution for injection, or in a dry lyophilized powder to be added to the formulation of the replacement enzyme during or immediately after reconstitution to prevent enzyme aggregation in vitro prior to administration.

When the PC (*e.g.,* migalastat or salt thereof) is formulated for oral administration, the tablets or capsules can be prepared by conventional means with pharmaceutically acceptable excipients such as binding agents (*e.g.,* pregelatinized maize starch, polyvinylpyrrolidone or hydroxypropyl methylcellulose); fillers (*e.g.,* lactose, microcrystalline cellulose or calcium hydrogen phosphate); lubricants (*e.g.,* magnesium stearate, talc or silica); disintegrants (*e.g.,* potato starch or sodium starch glycolate); or wetting agents (*e.g.,* sodium lauryl sulfate). The tablets may be coated by methods well known in the art. Liquid preparations for oral administration may take the form of, for example, solutions, syrups or suspensions, or they may be presented as a dry product for constitution with water or another suitable vehicle before use. Such liquid preparations may be prepared by conventional means with pharmaceutically acceptable additives such as suspending agents (*e.g.,* sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p- hydroxybenzoates or sorbic acid). The preparations may also contain buffer salts, flavoring, coloring and sweetening agents as appropriate. Preparations for oral administration may be suitably formulated to give controlled release of the active chaperone compound.

The pharmaceutical formulations of the PC (*e.g.,* migalastat or salt thereof) suitable for parenteral/injectable use generally include sterile aqueous solutions (where water soluble), or dispersions and sterile powders for the extemporaneous preparation of sterile injectable solutions or dispersion. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be brought about by various antibacterial and antifungal agents, for example, parabens, chlorobutanol, phenol, benzyl alcohol, sorbic acid, and the like. In many cases, it will be reasonable to include isotonic agents, for example, sugars or sodium chloride. Prolonged absorption of the injectable compositions can be brought about by the use in the compositions of agents delaying absorption, for example, aluminum monosterate and gelatin.

Sterile injectable solutions are prepared by incorporating the purified enzyme (if any) and the PC (*e.g.,* migalastat or salt thereof) in the required amount in the appropriate solvent with various of the other ingredients enumerated above, as required, followed by filter or terminal sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The formulation can contain an excipient. Pharmaceutically acceptable excipients which may be included in the formulation are buffers such as citrate buffer, phosphate buffer, acetate buffer, and bicarbonate buffer, amino acids, urea, alcohols, ascorbic acid, phospholipids; proteins, such as serum albumin, collagen, and gelatin; salts such as EDTA or EGTA, and sodium chloride; liposomes; polyvinylpyrollidone; sugars, such as dextran, mannitol, sorbitol, and glycerol; propylene glycol and polyethylene glycol (*e.g.,* PEG-4000, PEG-6000); glycerol; glycine or other amino acids; and lipids. Buffer systems for use with the formulations include citrate; acetate; bicarbonate; and phosphate buffers. Phosphate buffer is a preferred embodiment.

The route of administration of the chaperone compound may be oral or parenteral, including intravenous, subcutaneous, intra-arterial, intraperitoneal, ophthalmic, intramuscular, buccal, rectal, vaginal, intraorbital, intracerebral, intradermal, intracranial, intraspinal, intraventricular, intrathecal, intracisternal, intracapsular, intrapulmonary, intranasal, transmucosal, transdermal, or via inhalation.

Administration of the above-described parenteral formulations of the chaperone compound may be by periodic injections of a bolus of the preparation, or may be administered by intravenous or intraperitoneal administration from a reservoir which is external (*e.g.,* an i.v. bag) or internal (*e.g.,* a bioerodable implant).

Embodiments relating to pharmaceutical formulations and administration may be combined with any of the other embodiments of the invention, for example embodiments relating to methods of treating patients with classic Fabry disease, methods of treating ERT-naive patients with classic Fabry disease, methods of reducing kidney GL-3, methods of stabilizing renal function, methods of reducing LVM or LVMi, methods of reducing plasma lyso-Gb₃ and/or methods of treating gastrointestinal symptoms (*e.g.* diarrhea), methods of enhancing α-Gal A in a patient diagnosed with or suspected of having Fabry disease, use of a pharmacological chaperone for α-Gal A for the manufacture of a medicament for treating a patient diagnosed with Fabry disease or to a pharmacological chaperone for α-Gal A for use in treating a patient diagnosed with Fabry disease as well as embodiments relating to amenable mutations, the PCs and suitable dosages thereof.

In one or more embodiments, the PC (*e.g.,* migalastat or salt thereof) is administered in combination with ERT. ERT increases the amount of protein by exogenously introducing wild-type or biologically functional enzyme by way of infusion. This therapy has been developed for many genetic disorders, including lysosomal storage disorders such as Fabry disease, as referenced above. After the infusion, the exogenous enzyme is expected to be taken up by tissues through non-specific or receptor-specific mechanism. In general, the uptake efficiency is not high, and the circulation time of the exogenous protein is short. In addition, the exogenous protein is unstable and subject to rapid intracellular degradation as well as having the potential for adverse immunological reactions with subsequent treatments. In one or more embodiments, the chaperone is administered at the same time as replacement enzyme (*e.g*., replacement α-Gal A). In some embodiments, the chaperone is co-formulated with the replacement enzyme (*e.g.,* replacement α-Gal A).

In one or more embodiments, a patient is switched from ERT to migalastat therapy. In some embodiments, a patient on ERT is identified, the patient's ERT is discontinued, and the patient begins receiving migalastat therapy. The migalastat therapy can be in accordance with any of the methods described herein.

### Kidney GL-3

The dosing regimens described herein can reduce kidney GL-3 (*e.g.,* GL-3 inclusions per kidney interstitial capillary) in Fabry patients. As untreated Fabry patients typically exhibit an increase in kidney GL-3 over time, both reductions in and maintenance of kidney GL-3 are indications of a benefit of migalastat therapy. As described in further detail in the Example below, a Phase 3 study has found that migalastat therapy reduces kidney GL-3 in ERT-naive patients with classic Fabry disease. Accordingly, migalastat therapy can be used to treat classic Fabry patients by reducing and/or stabilizing kidney GL-3.

The migalastat therapy may reduce the increase in kidney GL-3 for a classic Fabry patient compared to the same patient without treatment with migalastat therapy. In one or more embodiments, the migalastat therapy provides a change in GL-3 inclusions per kidney interstitial capillary for a patient that is less than (*i.e.,* more negative than) 0, such as less than about -0.1, -0.2, -0.3, -0.4, -0.5, -0.6, -0.7, -0.8, -0.9 or -1. Expressed differently, in one or more embodiments, the migalastat therapy provides a reduction in GL-3 inclusions per kidney interstitial capillary of greater than 0, such as reductions of at least about 0.1, 0.2, 0.3, 0.4, 0.5, 0.6, 0.7, 0.8, 0.9 or 1.

In one or more embodiments, the migalastat therapy provides an average decrease in GL-3 inclusions per kidney interstitial capillary in a group of classic Fabry patients of at least about 0.1 after 6 months of administration of migalastat or a salt thereof. In various embodiments, the average decrease in the group of classic Fabry patients after 6 months of administration of migalastat or a salt thereof is at least about 0.1, 0.2, 0.3, 0.4, 0.5, 0.6, 0.7 or 0.8, such as about 0.8. In various embodiments, the classic Fabry patients are ERT-naive.

### Renal Function

The dosing regimens described herein can stabilize and/or enhance renal function (*e.g.,* eGFR) in Fabry patients. As untreated Fabry patients typically exhibit a deterioration of renal function over time, both enhancements in and maintenance of renal function are indications of a benefit of migalastat therapy. As described in further detail in the Example below, a Phase 3 study has found that migalastat therapy reduces stabilizes renal function in ERT-naive patients with classic Fabry disease. Accordingly, migalastat therapy can be used to treat classic Fabry patients by increasing and/or stabilizing renal function.

The migalastat therapy may arrest or decrease the reduction in renal function and/or increase renal function for a classic Fabry patient compared to the same patient without treatment with migalastat therapy. In one or more embodiments, the migalastat therapy provides an annualized change in eGFR_{CKD-EPI} for a patient that is greater than (*i*.*e*. more positive than) -5.0 mL/min/1.73 m²/year, such as greater than -4.5, -4.0, -3.5, -3.0, -2.5, -2.0, -1.5, -1.0, -0.9, -0.8, -0.7, -0.6, -0.5, -0.4, -0.3, -0.2, -0.1 or even greater than 0 mL/min/1.73 m²/year. In one or more embodiments, the migalastat therapy provides an annualized change in mGFRᵢₒₕₑₓₒₗ for a patient that is greater than -5.0 mL/min/1.73 m²/year, such as greater than -4.5, -4.0, -3.5, -3.0, -2.5, -2.0, -1.5, -1.0, -0.9, -0.8, -0.7, -0.6, -0.5, -0.4, -0.3, -0.2, -0.1 or even greater than 0 mL/min/1.73 m²/year. Accordingly, the migalastat therapy may reduce the decline or even improve the renal function of the patient. These annualized rates of change can be measured over a specific time period, such as over 6 months, 12 months, 18 months, 24 months, 30 months or 36 months.

In one or more embodiments, the migalastat therapy provides a mean annualized change in eGFR_{CKD-EPI} in a group of classic Fabry patients that is greater than -5.0 mL/min/1.73 m²/year, such as greater than -4.5, -4.0, -3.5, -3.0, -2.5, -2.0, -1.5, -1.0, -0.9, - 0.8, -0.7, -0.6, -0.5, -0.4, -0.3, -0.2, -0.1 or even greater than 0 mL/min/1.73 m²/year after 24 months of administration of migalastat or a salt thereof. In one or more embodiments, the migalastat therapy provides a mean annualized change in mGFRᵢₒₕₑₓₒₗ in a group of classic Fabry patients that is greater than -5.0 mL/min/1.73 m²/year, such as greater than -4.5, -4.0, - 3.5, -3.0, -2.5, -2.0, -1.5, -1.0, -0.9, -0.8, -0.7, -0.6, -0.5, -0.4, -0.3, -0.2, -0.1 or even greater than 0 mL/min/1.73 m²/year after 24 months of administration of migalastat or a salt thereof. In various embodiments, the classic Fabry patients are ERT-naive.

### Left Ventricular Mass

The dosing regimens described herein can improve LVM or LVMi in Fabry patients. The natural history of LVMi and cardiac hypertrophy in untreated Fabry patients regardless of phenotype (Patel, O'Mahony *et al.* 2015) is a progressive increase in LVMi between +4.07 and +8.0 g/m²/year (Kampmann, Linhart *et al.* 2008; Wyatt, Henley *et al.* 2012; Germain, Weidemann *et al.* 2013). As untreated Fabry patients typically exhibit an increase in LVMi over time, both decreases in and maintenance of LVMi are indications of a benefit of migalastat therapy. As described in further detail in the Example below, a Phase 3 study has found that migalastat therapy decreases LVMi in ERT-naive patients with classic Fabry disease. Accordingly, migalastat therapy can be used to treat classic Fabry patients by reducing LVM and/or reducing LVMi, including patients with LVH.

The migalastat therapy may reduce the increase in LVM or LVMi for a classic Fabry patient compared to the same patient without treatment with migalastat therapy. In one or more embodiments, the migalastat therapy provides a change in LVMi for a patient that is less than (*i.e.,* more negative than) 0 g/m², such as less than or equal to about -0.5, -1, -1.5, - 2, -2.5, -3, -3.5, -4, -4.5, -5, -5.5, -6, -7, -8, -9, -10, -11, -12, -13, -14, -15, -16, -17, -18, -19 or -20 g/m². Expressed differently, in one or more embodiments, the migalastat therapy provides a reduction in LVMi of greater than 0 g/m², such as reductions of at least about 0.5, 1, 1.5, 2, 2.5, 3, 3.5, 4, 4.5, 5, 5.5, 6, 7, 8, 9, 10, 11, 12, 13, 14, 15, 16, 17, 18, 19 or 20 g/m².

In one or more embodiments, the migalastat therapy provides an average decrease of LVMi in a group of classic Fabry patients of at least about 1 g/m² after 24 months of administration of migalastat or a salt thereof. In various embodiments, the average decrease in the group of classic Fabry patients after 24 months of administration of migalastat or a salt thereof is at least about 1, 2, 3, 4, 5, 6, 7, 8, 9, 10, 11, 12, 13, 14, 15, 16 or 17 g/m², such as about 16.7 g/m². In various embodiments, the classic Fabry patients are ERT-naïve.

### Plasma Lyso-Gb₃

The dosing regimens described herein can reduce plasma lyso-Gb₃ in Fabry patients. As untreated Fabry patients typically exhibit an increase in plasma lyso-Gb₃ over time, both reductions in and maintenance of plasma lyso-Gb₃ are indications of a benefit of migalastat therapy. As described in further detail in the Example below, a Phase 3 study has found that migalastat therapy reduces plasma lyso-Gb₃ in ERT-naive patients with classic Fabry disease. Accordingly, migalastat therapy can be used to treat classic Fabry patients by reducing and/or stabilizing plasma lyso-Gb₃.

The migalastat therapy may reduce the increase in plasma lyso-Gb₃ for a classic Fabry patient compared to the same patient without treatment with migalastat therapy. In one or more embodiments, the migalastat therapy provides a change in plasma lyso-Gb₃ for a patient that is less than (*i*.*e.*, more negative than) 0 nmol/L, such as less than about -5, -10, - 15, -20, -25, -30, -35 or -40 nmol/L. Expressed differently, in one or more embodiments, the migalastat therapy provides a reduction in plasma lyso-Gb₃ of greater than 0 nmol/L, such as reductions of at least about 5, 10, 15, 20, 25, 30, 35 or 40 nmol/L.

In one or more embodiments, the migalastat therapy provides an average decrease in plasma lyso-Gb₃ in a group of classic Fabry patients of at least about 5 nmol/L after 24 months of administration of migalastat or a salt thereof. In various embodiments, the average decrease in the group of classic Fabry patients after 6 months of administration of migalastat or a salt thereof is at least about 5, 10, 15, 20, 25, 30, 35 or 40 nmol/L, such as about 36 nmol/L. In various embodiments, the classic Fabry patients are ERT-naive.

### Gastrointestinal Symptoms

The dosing regimens described herein can treat gastrointestinal symptoms (e.g., diarrhea) in Fabry patients. As described in further detail in the Example below, a Phase 3 study has found that migalastat therapy reduces diarrhea symptoms in ERT-naive patients with classic Fabry disease. Accordingly, migalastat therapy can be used to treat classic Fabry patients by reducing gastrointestinal symptoms such as diarrhea.

The migalastat therapy may reduce the GSRS-D for a classic Fabry patient compared to the same patient without treatment with migalastat therapy. In one or more embodiments, the migalastat therapy provides a change in GSRS-D for a patient that is less than (*i.e.,* more negative than) 0, such as less than about -0.1, -0.2, -0.3, -0.4, -0.5, -0.6, -0.7, - 0.8, -0.9, -1, -1.1, -1.2, -1.3, -1.4, -1.5, -1.6, -1.7, -1.8, -1.9 or -2. Expressed differently, in one or more embodiments, the migalastat therapy provides a reduction in GL-3 inclusions per kidney interstitial capillary of greater than 0, such as reductions of at least about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9 or 2.

In one or more embodiments, the migalastat therapy provides an average decrease in GSRS-D in a group of classic Fabry patients of at least about 0.1 after 6 months of administration of migalastat or a salt thereof. In various embodiments, the average decrease in the group of classic Fabry patients after 6 months of administration of migalastat or a salt thereof is at least about 0.1, 0.15, 0.2, 0.25, 0.3, 0.35 or 0.4, such as about 0.4. In various embodiments, the classic Fabry patients are ERT-naïve.

In one or more embodiments, the migalastat therapy provides an average decrease in GSRS-D in a group of classic Fabry patients of at least about 0.1 after 24 months of administration of migalastat or a salt thereof. In various embodiments, the average decrease in the group of classic Fabry patients after 24 months of administration of migalastat or a salt thereof is at least about 0.1, 0.15, 0.2, 0.25, 0.3, 0.35, 0.4, 0.45, 0.5, 0.55, 0.6, 0.65, 0.7, 0.75, 0.8, 0.85, 0.9, 0.95 or 1, such as about 0.9. In various embodiments, the classic Fabry patients are ERT-naive.

### EXAMPLES

The compositions and processes of the present invention will be better understood in connection with the following examples, which are intended as an illustration only and not limiting of the scope of the invention. Various changes and modifications to the disclosed embodiments will be apparent to those skilled in the art and such changes and modifications including, without limitation, those relating to the processes, formulations and/or methods of the invention may be made without departing from the spirit of the invention and the scope of the appended claims.

### EXAMPLE 1: Dosing Regimens for the Treatment of Fabry Patients Using Migalastat Hydrochloride

This example describes a Phase 3 study of migalastat therapy in ERT-naive Fabry patients, including patients with classic Fabry disease.

***Patient Enrollment.*** Eligible patients were 16-74 years old and had genetically-confirmed Fabry disease; had either never received or had not received ERT for ≥6 months; had a *GLA* mutation that resulted in a mutant protein that would respond to migalastat, based on the HEK assay used at the time of enrollment; had an eGFR >30 ml/minute/1.73m², and had a urinary GL-3 ≥4 times the upper limit of normal.

***Study Design.*** Following eligibility-baseline assessments (2 months), patients were randomized to Stage 1-- 6 months of double-blind administration of 150 mg migalastat hydrochloride or placebo every other day. All patients completing Stage 1 were eligible to receive open-label migalastat in Stage 2 (months 6-12) and for an additional year (months 13-24) thereafter. The primary objective was to compare the effect of migalastat to placebo on kidney GL-3 as assessed by histological scoring of the number of inclusions in interstitial capillaries after 6 months of treatment. The secondary objectives of Stage 1 were to compare the effect of migalastat to placebo on urine GL-3 levels, on renal function, 24-hours urinary protein, and on safety and tolerability. The tertiary objectives were cardiac function, patient-reported outcomes, exploratory kidney analyses, and white blood cell α-Gal A activity. Study completers were eligible to enroll in the open-label extension study for up to 5 years.

***Kidney Histology Assessment.*** Each patient underwent a baseline kidney biopsy, as well as repeat kidney biopsies at 6 and 12 months. The number of GL-3 inclusions per kidney interstitial capillary per patient at baseline, and at 6 and 12 months was quantitatively assessed in 300 capillaries by 3 independent pathologists blinded to treatment and visit. All values for each individual biopsy at a given time were averaged prior to statistical analysis.

GL-3 changes in podocytes, endothelial cells, and mesangial cells, and glomerular sclerosis, were assessed qualitatively by the same 3 pathologists blinded to treatment/visit.

***Globotriaosylceramide and Globotriaosylsphingosine.*** Plasma lyso-Gb3 and 24-hour urine GL-3 were analyzed by liquid chromatography-mass-spectroscopy using a novel stable isotope-labeled internal standard, 13C6-lyso-Gb3 (lower-limit-of-quantification: 0.200 ng/mL, 0.254 nmol/L).

***Renal Function Assessment.*** Annualized rates of change (mL/min/1.73m²/year) were calculated using Chronic Kidney Disease Epidemiology Collaboration-eGFR_{CKD-EPI}) and measured iohexol clearance-mGFRᵢₒₕₑₓₒₗ).

***Echocardiography.*** LVMi, left posterior wall thickness, diastolic, interventricular septum thickness, diastolic and other parameters were assessed through blinded, centralized evaluation.

***Patient-Reported Outcomes.*** Patient-reported outcomes were assessed using the Gastrointestinal-Symptoms-Rating-Scale (GSRS), Short Form-36v2TM and Brief-Pain-Inventory-Pain-Severity-Component.

***Safety Analysis and Adverse Events.*** Randomized patients receiving ≥1 dose were included in the safety analysis, which comprised vital signs, physical exams, electrocardiograms, clinical labs, and adverse events.

***Statistical Analyses for Kidney Interstitial Capillary GL-3 Substrate.*** The primary Stage 1 (6 month) endpoint (ITT population with baseline biopsies, n=64) was the proportion of patients in the migalastat and placebo groups with a ≥50% reduction in GL-3 inclusions per kidney interstitial capillary. Two other Stage 1 endpoints were assessed (modified-ITT population: randomized patients with paired baseline and month 6 biopsies; n=60): percent change in GL-3 inclusions per kidney interstitial capillary, and percent of interstitial capillaries with zero GL-3 inclusions.

Efficacy analyses for GL-3 inclusions per kidney interstitial capillary and other pre-specified endpoints in Stage 2 (months 6-12) and the open-label-extension (months 12-24) were based on the modified intention to treat (mITT) - population consisting of randomized patients with mutant α-Gal A enzyme shown to be suitable for migalastat treatment by the validated assay; n=50).

For all outcomes except GL-3 inclusions per kidney interstitial capillary, data are pooled for patients within phenotype subgroups regardless of treatment allocation for the first six months (migalastat or placebo).

### Results

**Baseline Characteristics.** Sixty-seven patients (16-74 years-old; 64% female) with potentially responsive mutant α-Gal A were randomized (ITT population). Table 2 provides the baseline characteristics for the 50 patients in the ITT population with suitable mutant α-Gal A. There were no statistically significant differences in baseline parameters.

**Table 2: Baseline Characteristics**

| **Parameter** | | **Treatment Group** | | **Total** (N=50) |
|---|---|---|---|---|
| | | **Migalastat HCl** (N=28) | **Placebo to Migalastat HCl** (N=22) | |
| **Age** (**year**) (**n**) | | 28 | 22 | 50 |
| | Mean±SD | 41.5±13 | 45.1±8.0 | 43.1±11 |
| | Median | 37.0 | 45.5 | 45.0 |
| **Weight (kg) (n)** | | 28 | 22 | 50 |
| | Mean±SD | 72.6±15.35 | 76.1±16.52 | 74.1±15.81 |
| | Median | 72.3 | 74.0 | 72.8 |
| | **Number of Years of Diagnosis of Fabry Disease (n)** | 28 | 21 | 49 |
| | Mean±SD | 5.6±6.89 | 7.3±8.80 | 6.3±7.73 |
| | Median | 4.1 | 4.1 | 4.1 |
| **Number of patients previously on ERT (>6 months prior to baseline) (%)** | | 4 (14.3%) | 7 (31.8%) | 11 (22.0%) |

| **Use of ACEi/ARB/Ri at Baseline** | | | | |
|---|---|---|---|---|
| | Yes (%) | 9 (32.1%) | 12 (54.5%) | 21 (42.0%) |
| | No (%) | 19 (67.9%) | 10 (45.5%) | 29 (58.0%) |
| **Proteinuria >150mg/24h** (%) | | 17 (60.7%) | 18 (81.8%) | 35 (70.0%) |
| **Proteinuria >300mg/24h** (%) | | 8 (28.6%) | 11 (50.0%) | 19 (38.0%) |
| **Proteinuria >1000mg/24h** (%) | | 3 (10.7%) | 3 (13.6%) | 6 (12.0%) |
| **mGFR _{Iohexol} (mL/min/1.73m²)** (**n)** | | 27 | 21 | 48 |
| | Mean±SD | 79.95±30.9 | 83.12±22.8 | 81.34±27.5 |
| | Median | 84.90 | 82.20 | 83.40 |
| **eGFR_{CKD-EPI}** (**mL/min/1.73m²**) | | 28 | 22 | 50 |
| | Mean±SD | 94.4±27.0 | 90.6±17.1 | 92.7±23.0 |
| | Median | 96.6 | 93.5 | 94.0 |
| **Lyso-Gb₃** (**n**) | | 18 | 13 | 31 |
| | Mean (nmol/L) ±SD | 47.3±62 | 41.9±39 | 45.0±53 |

Published reports of clinical phenotype(s) associated with the genotypes of patients with suitable mutations (n=50) indicate that 30 (60%) had mutations associated with the classic phenotype of Fabry disease, one (2%) with the non-classic phenotype, three (6%) with both phenotypes, and 16 (32%) not yet classified. Residual WBC α-Gal A activity <3% was found in 14 of 16 (87%) males; 29 of 31 (94%) males and females had elevated plasma lyso-Gb3, and 47 of 50 (94%) males and females had multi-organ system disease.

Male patients were identified as having classic Fabry disease based on multiorgan system involvement and PBMC α-Gal A activity <3% of normal. Classic male patients (n=14) had more severe manifestations of Fabry disease at baseline compared with other patients (i.e., male patients with the non-classic phenotype and female patients; n=36) (FIGS. 4A-4E). Mean (standard deviation [SD]) baseline eGFR_{CKD-EPI} (87.8 [8.98] vs. 95.3 [3.37] mL/min/1.73 m²) and mGFRᵢₒₕₑₓₒₗ (78.6 [6.90] vs. 88.2 [3.95] mL/min/1.73 m²) were lower in classic male patients than other patients and LVMi was higher (114.3 [7.31] vs. 88.2 [5.90] g/m²). The mutations in the classic male subgroup were: p.Ile253Thr (n=2), p.Pro259Arg (n=2), p.Gly183Asp, p.Leu243Phe, p.Cys174Arg, p.Asp55Val/Gln57Leu, p.Gly144Val, p.Arg301Gln, p.Gly373Ser, p.Asp322Glu, p.Gly325Arg, and p.Tyr216Cys (n=1 each).

***In Vitro* Activity..** Table 3 below provides the baseline PBMC α-Gal A activity and the effect of migalastat on α-Gal A activity as measured according to the HEK assay.

**Table 3: Effect of on α-Gal A Activity**

| | | Measured in PBMC at Baseline^{a} | | Measured in HEK-293 Cell Lysate | | | | | |
|---|---|---|---|---|---|---|---|---|---|
| α-Gal A mutant form | | -Migalastat | | -Migalastat | | +Migalastat | | Absolute increase at 10 µM (%WT) | α-Gal A activity at 10 µm (fold over baseline) |
| Amino acid change | Nucleotide change | α-Gal A (nmol/mg/hr) | % WT | α-Gal A activity (nmol/mg/hr) | % WT | α-Gal A activity (nmol//mg/hr)^{†} | % WT | | |
| *GLA mutations found in patients meeting criteria for classic phenotype^{b}* | | | | | | | | | |
| p.Asp55Val/ Gln57Len | c.164 A>T; c.170 A>T | BLQ | 0.0 | BLD | N/A | 2526 ± 287* | 8.0 ± 0.5 | 8.0 | NC |
| p.Gly144Val | c.431 G>T | 0.05 | 0.2 | 253 ± 21 | 0.8 ± 0.1 | 2924 ± 335* | 9.2 ± 1.2 | 8.4 | 11.58 |
| p.Cys174Arg | c.520 T>C | 0.28 | 1.3 | 4671 ± 270 | 14.3 ± 0.6 | 16505 ± 393* | 51.6 ± 2.0 | 37.4 | 3.53 |
| p.Gly183Asp | c.548 G>A | 0.04 | 0.2 | 207 ± 15 | 0.7 ± 0.1 | 6074 ± 212* | 19.1 ± 1.0 | 18.4 | 29.31 |
| p.Tyr216Cys | c.647 A>G | BLQ | 0.0 | 673 ± 38 | 2.0 ± 0.1 | 7003 ± 305* | 20.7 ± 0.8 | 18.7 | 10.40 |
| p.Leu243Phe | c.729 G>C | 0.19 | 0.9 | 2694 ± 117 | 7.9 ± 0.3 | 14370 ± 618* | 42.3 ± 1.4 | 34.4 | 5.33 |
| p.Ile253Thr | c.758 T>C | 0.63/0.58 | 2.9/2.6 | 11287 ± 506 | 38.9 ± 3.0 | 23417 ± 1077* | 80.2 ± 5**.**9 | 41.3 | 2.07 |
| p.Pro259Arg | c.776 C>G | 0.44/0.6 | 2.0/2.7 | 6681 ± 364 | 23.3 ± 2.3 | 17645 ± 515* | 60.3 ± 3.8 | 37.0 | 2.64 |
| p.Arg301Gln | c.902 G>A | 0.42 | 1.9 | 1914 ± 52 | 5.5 ± 0.2 | 15547 ± 353* | 44.5 ± 1.0 | 39.0 | 8.12 |
| p.Asp322Glu | c.966 C>A | 0.2 | | 2398 ± 141 | 6.7 ± 0.2 | 9554 ± 667 | 26.8 ± 1.3 | 20.0 | 3.98 |
| p.Gly325Arg | c.973G>C | 0.07 | 0.3 | 909 ± 31 | 2.6 ± 0.1 | 9244 ± 417***** | 26.6 ± 1.4 | 24.0 | 10.17 |
| p.Gly373Ser | c.1117 G>A | 0.29 | 1.3 | 1544 ± 69 | 4.8 ± 0.3 | 5128 ± 288* | 15.7 ± 0.8 | 10.9 | 3.32 |

| ***GLA mutations found in males that did not meet criteria for classic phenotype*** | | | | | | | | | |
|---|---|---|---|---|---|---|---|---|---|
| p.Asp33Gly | c.98 A>G | 1.42 | 6.5 | 9913 ± 600 | 29.3 ± 1.8 | 24033 ± 865* | 70.6 ± 2.4 | 41.3 | 2.42 |
| p.Ala156Thr | c.466 G>A | 9.05 | 41.1 | 907 ± 31 | 2.8 ± 0.1 | 7034 ± 289* | 21.9 ± 0.9 | 19.1 | 7.75 |
| p.Asp244Asn | 730 G>A | 13.17 | 59.8 | 10317 ± 386 | 30.9 ± 1.6 | 16321 ± 402* | 48.7 ± 1.7 | 17.8 | 1.58 |
| p.Arg356Trp | c.1066 C>T | 1.22 | 5.5 | 3526 ± 240 | 11.0 ± 07 | 15570 ± 830* | 49.1 ± 2.6 | 38.1 | 4.42 |

| ***GLA mutations in female patients*** | | | | | | | | | |
|---|---|---|---|---|---|---|---|---|---|
| p.Leu36Trp | c.107 T>G | - | - | 241 ± 19 | 0.7 ± 0.1 | 5182 ± 463* | 16.6 ± 2.1 | 15.9 | 21.49 |
| p.Gly85Asp | c.254 G>A | - | - | 888 ± 38 | 2.7 ± 0.1 | 4534 ± 135* | 14.3 ± 0.9 | 11.6 | 5.10 |
| p.Arg112His | c.335 G>A | - | - | 845 ± 39 | 2.6 ± 0.1 | 5583 ± 215***** | 17.4 ± 0.8 | 14.8 | 6.61 |
| p.Met187Ile | c.561 G>A | - | - | 1775 ± 57 | 5.1 ± 0.2 | 10824 ± 555* | 30.7 ± 1.1 | 25.6 | 6.10 |
| p.Pro205Thr | c.613 C>A | - | - | 4802 ± 230 | 14.4 ± 0.9 | 16371 ± 647***** | 48.8 ± 2.2 | 34.4 | 3.41 |
| p.Gly258Arg | c.772 G**>**C | - | - | 9558 ± 348 | 32.6 ± 2.1 | 22630 ± 801* | 78.1 ± 5.3 | 45.5 | 2.37 |
| p.Leu300Pro | c**.**899 T>C | - | - | 1277 ± 38 | 3.7 ± 01 | 13219 ± 412* | 37.9 ± 1.2 | 34.2 | 10.35 |
| p.Pro293Thr | c.877 C>A | - | - | 229 ± 20 | 0.7 ± 0.1 | 4488 ± 327* | 13.3 ± 1.1 | 12.7 | 19.60 |
| p.Phe 295Cys | c.884 T>G | - | - | 1196 ± 38 | 3.4 ± 0.1 | 5051 ± 190* | 14.5 ± 0.6 | 11.1 | 4.22 |
| p.Gly271Ser; Asp313Tyr | c.811 G>A; c.937 G>T | - | - | BLD | N/A | 877 ± 18* | 3.0 ± 0.2 | 3.0 | NC |
| p.Ile317Thr | c.950 T>C | - | - | 2298 ± 338 | | 6.5 ± 0.6 7812 ± 530* | 23.6 ± 1.0 | 17.0 | 3.40 |
| p**.**Asp264Tyr | c.790 G>T | - | - | 143 ± 13 | 0.5 ± 0.0 | 1842 ± 100* | 6.2 ± 0.3 | 5.7 | 12.89 |
| p.Gly260Ala | c.779 G>C | - | - | 2221 ± 142 | 7.5 ± 0.6 | 10749 ± 403* | 37.4 ± 3.1 | 29.9 | 4.84 |
| p.Met284Thr | c.851 T>C | - | - | 606 ± 40 | 1.7 ± 0.1 | 5050 ± 268* | 14.3 ± 0.6 | 12.6 | 8.33 |
| p.Gly183Asp | c.548 G>A | - | - | 207 ± 15 | 0.7 ± 0.1 | 6074 ± 212* | 19.1 ± 1.0 | 18.4 | 29.31 |
| p.Arg301Gln | c.902 G>A | - | - | 1914 ± 52 | 5.5 ± 0.2 | 15547 ± 353* | 44.5 ± 1.0 | 39.0 | 8.12 |
| p.Ile253Thr | c.758 T>C | - | - | 11287 ± 506 | 38.9 ± 3.0 | 23417 ± 1077* | 80.2 ± 5.9 | 41.3 | 2.07 |
| p**.**Asp322Glu | c.966 C>A | - | - | 2398 ± 141 | 6.7 ± 0.2 | 9554 ± 667* | 26.8 ± 1.3 | 20.0 | 3.98^{.} |

| | | **Measured in PBMC at Baseline^{a}** | | **Measured in HEK-293 Cell Lysate** | | | | | |
|---|---|---|---|---|---|---|---|---|---|
| **α-Gal A mutant form** | | **-Migalastat** | | **-Migalastat** | | | | **Absolute increase at 10 µM (%WT)** | **α-Gal A activity at 10 µM (fold over baseline)** |
| **Amino acid change** | **Nucleotide change** | **α-Gal A activity (nmol/mg/hr)** | **% wr** | **α-Gal A (nmol/mg/hr)** | **% WT** | **α-Gal A activity (nmol/mg/hr)** | **% WT** | | |
| p.Ile270Thr | c.809 T>C | -- | -- | 1846 ± 119 | 6.3 ± 0.5 | 12416 ± 377* | 42.8 ± 3.0 | 36.5 | 6.73 |
| p.Gly325Arg | c.973G>C | -- | -- | 909 ± 31 | 2.6 ± 0.1 | 9244 ± 417* | 26.6 ± 1.4 | 24.0 | 10.17 |
| P.Tyr216Cys | c.647 A>G | -- | -- | 673 ± 38 | 2.0 ± 0.1 | 7003 ± 305* | 20.7 ± 0.8 | 11.7 | 10.40 |
| p.Pro259Arg | c.776 C>G | -- | -- | 6681 ± 364 | 23.3 ± 2.3 | 17645 ± 515* | 60.3 ± 3.8 | 37.0 | 2.64 |
| p.Met284Thr | c.851 T>C | -- | -- | 606 ± 40 | 1.7 ± 0.1 | 5050 ± 268* | 14.3 ± 0.6 | 12.6 | 8.33 |
| p.Gly258Arg | c.772 G>C | -- | -- | 9558 ± 348 | 32.6 ± 2.1 | 22630 ± 801* | 78.1 ± 5.8 | 45.5 | 2.37 |

| | | | | | | | | | |
|---|---|---|---|---|---|---|---|---|---|
| BLD = below the limit of detection (<142 nmol/mg/hr); PBMC = peripheral blood mononuclear cells; SEM = standard error of the mean; WT = wild type. Data are expressed as the mean ± SEM of twenty data points: mutant α-Gal A activity is expressed as a percentage of the α-Gal A activity measured in WT cell lysates incubated without migalastat (-Migalastat) assayed in parallel. Absolute increase at 10 µM (%WT) = the percentage of wild-type α-Gal A activity with 10 µM migalastat (+Migalastat) minus the baseline (-Migalastat) percentage of wild-type α-Gal A activity. α -Gal A activity at 10 µM (fold over baseline) = α-Gal A activity in mutant-transfected cell lysate with 10 µM migalastat / α-Gal A activity in mutant-transfected cell lysate without migalastat. Statistically significant differences in α-Gal A activity without migalastat (n=20) versus with 10 uM migalastat (n=20) were determined using a one-tailed Mann-Whitney U non-parametric test: *p < 0.001. Asterisks indicate a Gal A mutant forms that show a statistically significant increase in α Gal A activity after incubation with 10 uM migalastat. "BLD" indicates that the mean a Gal A activity (mean of n=20) was below the limit of detection (<142 nmol/mg/hr; value is equal to 3 * standard deviation of the pcDNA activity in nmol/mg/hr after vector subtraction across 128 samples assayed in ten method validation experiments). ^{a}PBMC date are only shown for male patients. ^{b}Criteria for classic phenotype: male, PBMC α-Gal A activity <3% of normal multi-organ disease. | | | | | | | | | |

For the *GLA* mutations found in patients enrolled in this study which have been reported as associated with the classic phenotype in the literature, the mean absolute increase in α-Gal A activity with 10 µM of migalastat was 24.8% of WT, and mean activity increased by 8.2-fold over baseline. For all other mutations, the mean absolute increase in α-Gal A activity was 24.5% of WT, and mean activity increased by 6.8-fold over baseline
**Migalastat and Renal Function.** The mean (standard deviation [SD]) annualized rate of change in eGFR_{CKD-EPI} from baseline (or month 6 for patients randomized to placebo) to month 24 was -0.3 (3.76) mL/min/1.73 m² (95% CI -2.80, 2.25) in the classic male subgroup and -0.3 (4.47) mL/min/1.73 m² (95% CI 1.98, 1.48) in the other patients subgroup (FIG. 4A). Over this time, median annualized rates of change in classic males and other patients were 0.25 mL/min/BSA (min, max: -8.6, 4.3) and 0.33 mL/min/BSA (min, max: -13.8, 7.4), respectively. Mean (SD) annualized rate of change in mGFRᵢₒₕₑₓₒₗ over the same time period was -3.0 (6.04) mL/min/1.73 m² (95% CI -7.65, 1.64) in classic males and - 1.0 (6.66) mL/min/1.73 m² (95% CI -4.39, 2.33) in other patients. There was one classic male patient who had a reduction of -16 mg/ml 71.73 m² from month 6 to month 24. Median (range) change in mGFRᵢₒₕₑₓₒₗ from baseline to month 24 was -1.03 mg/mL/1.73 m² (min, max: -16.2, 3.0) in classic males and -0.34 mg/mL/1.73 m² (min, max: -20.4, 15.8) in other patients.

Results were similar to those for the overall amenable population in this study, in which migalastat stabilized GFR in patients with amenable mutations, with annualized changes from baseline to month 24 in eGFR_{CKD-EPI} and mGFRᵢₒₕₑₓₒₗ of -0.3 (0.66) and -1.5 (1.33) mL/min/1.73 m², respectively. The use of angiotensin-converting enzyme (ACE) inhibitors and angiotensin receptor blockers (ARBs) did not change during the study, and therefore did not impact the renal function data.

**Migalastat and Cardiac Function.** Migalastat also led to reductions in LVMi in both subgroups. Of note, baseline LVMi was substantially higher in classic male patients than other patients (114.3 g/m² vs 88.2 g/m²), and seven (50%) classic male patients had LVH at baseline. Mean (SD) change from baseline (or month 6) to month 24 in LVMi was - 16.7 (18.64) g/m² (95% CI -31.1, -2.4) in classic males and -3.2 (18.66) g/m² (95% CI -12.5, 6.1) in other patients (FIG. 4B).

In comparison, the LVMi change in the overall migalastat-treated population in this study was -7.7 g/m² (95% CI, -15.4, -0.01) after 24 months of treatment with migalastat, with a greater decrease in patients with LVH (-18.6 g/m² [95% CI, -38.2, 1.0]).

Moreover, in a comparison with published data for males with a classic phenotype, the results of this analysis indicate that patients treated with migalastat had better GFR and LVMi outcomes than patients who do not receive treatment. Studies of untreated males reported an annualized change from baseline in eGFR (measured using eGFR_{EKD-EPI} or eGFR_{MDRD}) ranging from -2.6 to -12.7 mL/min/1.73 m². For LVMi, studies of untreated males reported annualized increases ranging from 4.1 to 8.0 g/m². Furthermore, migalastat appears to have potential beneficial effects compared with ERT in this population of patients. While ERT has been shown to improve LVMi in young patients (age 18-29 years), the impact of migalastat was observed across the age spectrum, with 7/8 classic male patients (age range 16-61 years; mean 42.4 years) with data reporting a reduction in LVMi.

**Migalastat and Gastrointestinal Signs and Symptoms.** Mean (SD) GSRS-D scores at baseline were 2.4 (1.69) in classic male patients and 2.1 (1.49) in other patients. Eight (57%) classic male patients had diarrhea symptoms at baseline (i.e., GSRS-D score ≥1). In classic male patients, six months of treatment with migalastat improved scores in GSRS-D (mean [SD] change from baseline -0.3 [1.75]), whereas patients treated with placebo did had a small increase (0.2 [0.46]). Classic male patients benefited from continuing treatment with migalastat or from switching from placebo to migalastat. By 24 months, the mean (SD) change from baseline (or month 6) in diarrhea symptoms was -0.9 (1.75) (FIG. 4C), with 7 of the 8 (88%) classic male patients with diarrhea at baseline achieving a minimal clinically important difference (MCID) reduction of 0.33 in GSRS-D. The corresponding change in other patients was -0.5 (1.01) at month 24. These results are similar to those of the total amenable patient population in this study, in which patients with amenable mutations treated with migalastat had mean changes in GSRS-D of -0.3 and -0.5 at month 6 and month 24, respectively.

**Migalastat and Kidney GL-3.** During the first 6 months of the study, the mean (SD) number of GL-3 inclusions per kidney interstitial capillary decreased from baseline in classic males treated with migalastat (-0.8 [-0.78]) (FIG. 4D). Conversely, GL-3 inclusions increased (mean [SD], 0.3 [0.94]) during that time period in classic male patients receiving placebo. Upon switching from placebo to migalastat at month 6, GL-3 inclusions per kidney interstitial capillary decreased by a mean (SD) of -0.7 (0.91) over the next 6 months in these patients. These results mirror those reported for the total amenable patient population in this study, in which GL-3 inclusions decreased over 6 months of migalastat treatment (mean, -0.25) and increased (mean, 0.07) over 6 months of placebo treatment. In the total patient population, a subsequent decrease in mean GL-3 inclusions of -0.33 was seen upon switching from placebo to migalastat at month 6.

Decreases in mean (SD) number of GL-3 inclusions per kidney interstitial capillary were seen in the other patient subgroup whether treated with migalastat from month 0-6 (-0.1 [0.30]) or switched from placebo to migalastat at month 6 (change from month 6-12; -0.1 [0.24]) (FIG. 4D). A small decrease was also noted in the other patient subgroup treated with placebo from month 0-6 (-0.05 [0.10]), and although overall decreases were small, likely due to the low baseline level, the magnitude of change with migalastat was twice that of placebo.

**Migalastat and Plasma Lyso-Gb₃.** Migalastat was associated with a reduction in plasma lyso-Gb₃ in both classic male and other patient subgroups. The reduction was more than twice as large in patients with the classic phenotype, probably due to the higher baseline level compared with the other patient subgroup mean [SD], 99.8 [35.28] vs. 29.3 [48.32] nmol/L). The average (SD) change from baseline (or month 6) to month 24 in plasma lyso-Gb₃ was -36.04 (34.48) nmol/L (95% CI -67.93, -4.15) for classic males and -16.33 (19.73) nmol/L (95% CI -25.07, -7.58) for other patients (FIG. 4E).

**Safety and Adverse Events.** During Stage 1, the treatment-emergent adverse events were similar between groups. Adverse events with a higher frequency in patients receiving migalastat compared to placebo were headache (12/34 patients-35% versus 7/33 patients-21%) and nasopharyngitis (6/34 patients-18% versus 2/34-6%). The most frequently reported adverse events for Stage 2 were headache (9/63 patients-14%) and procedural pain (7/63 patients-11%-related to kidney biopsies) and, for the open-label-extension, proteinuria (9/57 patients-16%), headache (6/57 patients-11%), and bronchitis (6/57 patients-11%). Most adverse events were mild or moderate in severity. No adverse events led to migalastat discontinuation.

Six patients experienced serious adverse events during Stage 1 (2: migalastat; 4: placebo), 5 during Stage 2, and 11 during the open-label-extension. Two serious adverse events were assessed as possibly related to migalastat by the investigator-- fatigue and paresthesia. Both occurred in the same patient between months 12-24 and resolved. No individual serious adverse event was reported by >1 patient. Two patients discontinued migalastat due to serious adverse events; both were deemed unrelated to migalastat. No deaths were reported.

Treatment-emergent proteinuria was reported in 9 patients (16%) between months 12-24, and in one case, was judged as migalastat-related. In 5 patients, the 24-month values were in the same range as baseline. Three patients with suitable mutations had overt baseline proteinuria (>1g/24-hr), which increased over 24 months. In 23/28 patients with baseline proteinuria <300 mg/24-h, 24-hour urine protein remained stable during migalastat treatment.

There was no progression to end-stage renal disease, no cardiac death and no stroke as defined in Banikazemi et al.. There was a single case of transient ischemic attack-judged unrelated to migalastat.

Analyses of vital sign, physical findings, laboratory, and ECG parameters did not reveal any clinically relevant effect of migalastat.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. All other published references, documents, manuscripts and scientific literature cited herein are hereby incorporated by reference.

While this invention has been particularly shown and described with references to preferred embodiments thereof, it will be understood by those skilled in the art that various changes in form and details may be made therein without departing from the scope of the invention encompassed by the appended claims.

**The present invention is also exemplified by the following preferred embodiments:**
1. A method of reducing kidney globotriaosylceramide (GL-3) in a patient having classic Fabry disease, the method comprising administering to the patient an effective amount of migalastat or salt thereof at a frequency of once every other day for reducing the patient's kidney GL-3, wherein the effective amount is about 100 mg to about 150 mg free base equivalent (FBE).
2. The method of **item** 1, wherein the patient has elevated kidney interstitial capillary GL-3 prior to initiating the administration of the migalastat or salt thereof.
3. The method of **item** 1 or 2, wherein reducing kidney GL-3 comprises reducing GL-3 inclusions per kidney interstitial capillary.
4. The method of any one of **items** 1-3, wherein the patient has a mutation in α-Gal A selected from the group consisting of I253T, P259R, G183D, L243F, C174R, D55V/Q57L, G144V, R301Q, G373S, D322E, G325R and Y216C.
5. The method of any one of **items** 1-4, wherein the migalastat or salt thereof enhances α-galactosidase A activity.
6. The method of any one of **items** 1-5, wherein the effective amount is about 123 mg FBE.
7. The method of any one of **items** 1-6, wherein the effective amount is about 123 mg of migalastat free base.
8. The method of any one of **items** 1-6, wherein the salt of migalastat is migalastat hydrochloride.
9. The method of **item** 8, wherein the effective amount is about 150 mg of migalastat hydrochloride.
10. The method of any one of **items** 1-9, wherein the migalastat or salt thereof is in an oral dosage form.
11. The method of **item** 10, wherein the oral dosage form comprises a tablet, a capsule or a solution.
12. The method of any one of **items** 1-11, wherein the migalastat or salt thereof is administered for at least 6 months.
13. The method of any one of **items** 1-12, wherein administration of the migalastat or salt thereof to a group of classic Fabry patients provides an average decrease in GL-3 inclusions per kidney interstitial capillary of at least about 0.5 after 6 months of the administration of the migalastat or salt thereof.
14. A method of treating classic Fabry disease in a patient in need thereof, the method comprising administering to the patient an effective amount of migalastat or salt thereof at a frequency of once every other day, wherein the effective amount is about 100 mg to about 150 mg free base equivalent (FBE) and wherein administering the migalastat or salt thereof reduces kidney globotriaosylceramide (GL-3) in the patient.
15. The method of **item** 14, wherein the patient has elevated kidney interstitial capillary GL-3 prior to initiating the administration of the migalastat or salt thereof.
16. The method of **item** 14 or 15, wherein reducing kidney GL-3 comprises reducing GL-3 inclusions per kidney interstitial capillary.
17. The method of any one of **items** 14-16, wherein the patient has a mutation in α-Gal A selected from the group consisting of I253T, P259R, G183D, L243F, C174R, D55V/Q57L, G144V, R301Q, G373S, D322E, G325R and Y216C.
18. The method of any one of **items** 14-17, wherein the migalastat or salt thereof enhances α-galactosidase A activity.
19. The method of any one of **items** 14-18, wherein the effective amount is about 123 mg FBE.
20. The method of any one of **items** 14-19, wherein the effective amount is about 123 mg of migalastat free base.
21. The method of any one of **items** 14-19, wherein the salt of migalastat is migalastat hydrochloride.
22. The method of **item** 20, wherein the effective amount is about 150 mg of migalastat hydrochloride.
23. The method of any one of **items** 14-22, wherein the migalastat or salt thereof is in an oral dosage form.
24. The method of **item** 23, wherein the oral dosage form comprises a tablet, a capsule or a solution.
25. The method of any one of **items** 14-24, wherein the migalastat or salt thereof is administered for at least 6 months.
26. The method of any one of **items** 14-25, wherein administration of the migalastat or salt thereof to a group of classic Fabry patients of provides an average decrease in GL-3 inclusions per kidney interstitial capillary of at least about 0.5 after 6 months of the administration of the migalastat or salt thereof.
27. A method of stabilizing renal function in a patient having classic Fabry disease, the method comprising administering to the patient an effective amount of migalastat or salt thereof at a frequency of once every other day for stabilizing the patient's renal function, wherein the effective amount is about 100 mg to about 150 mg free base equivalent (FBE).
28. The method of **item** 27, wherein the patient has renal impairment prior to initiating the administration of the migalastat or salt thereof.
29. The method of **item** 27 or 28, wherein the patient has a mutation in α-Gal A selected from the group consisting of I253T, P259R, G183D, L243F, C174R, D55V/Q57L, G144V, R301Q, G373S, D322E, G325R and Y216C.
30. The method of any one of **items** 27-29, wherein the migalastat or salt thereof enhances α-galactosidase A activity.
31. The method of any one of **items** 27-30, wherein the effective amount is about 123 mg FBE.
32. The method of any one of **items** 27-31, wherein the effective amount is about 123 mg of migalastat free base.
33. The method of any one of **items** 27-31, wherein the salt of migalastat is migalastat hydrochloride.
34. The method of **item** 33, wherein the effective amount is about 150 mg of migalastat hydrochloride.
35. The method of any one of **items** 27-34, wherein the migalastat or salt thereof is in an oral dosage form.
36. The method of **item** 35, wherein the oral dosage form comprises a tablet, a capsule or a solution.
37. The method of any one of **items** 27-36, wherein the migalastat or salt thereof is administered for at least 6 months.
38. The method of any one of **items** 27-37, wherein administration of the migalastat or salt thereof to a group of classic Fabry patients provides a mean annualized rate of change in eGFR_{CKD-EPl} of greater than -1.0 mL/min/1.73 m² after 24 months of the administration of the migalastat or salt thereof.
39. A method of treating classic Fabry disease in a patient in need thereof, the method comprising administering to the patient an effective amount of migalastat or salt thereof at a frequency of once every other day, wherein the effective amount is about 123 mg free base equivalent (FBE) and wherein administering the migalastat or salt thereof stabilizes renal function in the patient.
40. The method of **item** 39, wherein the patient has renal impairment prior to initiating the administration of the migalastat or salt thereof.
41. The method of **item** 39 or 40, wherein the patient has a mutation in α-Gal A selected from the group consisting of I253T, P259R, G183D, L243F, C174R, D55V/Q57L, G144V, R301Q, G373S, D322E, G325R and Y216C.
42. The method of any one of **items** 39-41, wherein the migalastat or salt thereof enhances α-galactosidase A activity.
43. The method of any one of **items** 39-42, wherein the effective amount is about 123 mg FBE.
44. The method of any one of **items** 39-43, wherein the effective amount is about 123 mg of migalastat free base.
45. The method of any one of **items** 39-43, wherein the salt of migalastat is migalastat hydrochloride.
46. The method of **item** 45, wherein the effective amount is about 150 mg of migalastat hydrochloride.
47. The method of any one of **items** 39-46, wherein the migalastat or salt thereof is in an oral dosage form.
48. The method of **item** 47, wherein the oral dosage form comprises a tablet, a capsule or a solution.
49. The method of any one of **items** 39-48, wherein the migalastat or salt thereof is administered for at least 6 months.
50. The method of any one of **items** 39-49, wherein administration of the migalastat or salt thereof to a group of classic Fabry patients provides a mean annualized rate of change in eGFR_{CKD-EPl} of greater than -1.0 mL/min/1.73 m² after 24 months of the administration of the migalastat or salt thereof.
51. A method of reducing left ventricular mass (LVM) in a patient having classic Fabry disease, the method comprising administering to the patient an effective amount of migalastat or salt thereof at a frequency of once every other day for reducing the patient's LVM, wherein the effective amount is about 100 mg to about 150 mg free base equivalent (FBE).
52. The method of **item** 51, wherein the patient has left ventricular hypertrophy (LVH) prior to initiating administration of the migalastat or salt thereof.
53. The method of **item** 51 or 52, wherein reducing LVM comprises reducing left ventricular mass index (LVMi).
54. The method of any one of **items** 51-53, wherein the patient has a mutation in α-Gal A selected from the group consisting of I253T, P259R, G183D, L243F, C174R, D55V/Q57L, G144V, R301Q, G373S, D322E, G325R and Y216C.
55. The method of any one of **items** 51-54, wherein the migalastat or salt thereof enhances α-galactosidase A activity.
56. The method of any one of **items** 51-55, wherein the effective amount is about 123 mg FBE.
57. The method of any one of **items** 51-56, wherein the effective amount is about 123 mg of migalastat free base.
58. The method of any one of **items** 51-56, wherein the salt of migalastat is migalastat hydrochloride.
59. The method of **item** 58, wherein the effective amount is about 150 mg of migalastat hydrochloride.
60. The method of any one of **items** 51-59, wherein the migalastat or salt thereof is in an oral dosage form.
61. The method of **item** 60, wherein the oral dosage form comprises a tablet, a capsule or a solution.
62. The method of any one of **items** 51-61, wherein the migalastat or salt thereof is administered for at least 6 months.
63. The method of any one of **items** 51-62, wherein administration of the migalastat or salt thereof to a group of classic Fabry patients provides an average decrease in LVMi of at least about 5 g/m² after 24 months of the administration of the migalastat or salt thereof.
64. A method of treating classic Fabry disease in a patient in need thereof, the method comprising administering to the patient an effective amount of migalastat or salt thereof at a frequency of once every other day, wherein the effective amount is about 100 mg to about 150 mg free base equivalent (FBE) and wherein administering the migalastat or salt thereof reduces the patient's left ventricular mass (LVM).
65. The method of **item** 64, wherein the patient has left ventricular hypertrophy (LVH) prior to initiating administration of the migalastat or salt thereof.
66. The method of **item** 64 or 65, wherein reducing LVM comprises reducing left ventricular mass index (LVMi).
67. The method of any one of **items** 64-66, wherein the patient has a mutation in α-Gal A selected from the group consisting of I253T, P259R, G183D, L243F, C174R, D55V/Q57L, G144V, R301Q, G373S, D322E, G325R and Y216C.
68. The method of any one of **items** 64-67, wherein the migalastat or salt thereof enhances α-galactosidase A activity.
69. The method of any one of **items** 64-68, wherein the effective amount is about 123 mg FBE.
70. The method of any one of **items** 64-69, wherein the effective amount is about 123 mg of migalastat free base.
71. The method of any one of **items** 64-69, wherein the salt of migalastat is migalastat hydrochloride.
72. The method of **item** 71, wherein the effective amount is about 150 mg of migalastat hydrochloride.
73. The method of any one of **items** 64-72, wherein the migalastat or salt thereof is in an oral dosage form.
74. The method of **item** 73, wherein the oral dosage form comprises a tablet, a capsule or a solution.
75. The method of any one of **items** 64-74, wherein the migalastat or salt thereof is administered for at least 6 months.
76. The method of any one of **items** 64-75, wherein administration of the migalastat or salt thereof to a group of classic Fabry patients provides an average decrease in LVMi of at least about 5 g/m² after 24 months of the administration of the migalastat or salt thereof.
77. A method of reducing plasma globotriaosylsphingosine (lyso-Gb₃) in a patient having classic Fabry disease, the method comprising administering to the patient an effective amount of migalastat or salt thereof at a frequency of once every other day for reducing the patient's plasma lyso-Gb₃, wherein the effective amount is about 100 mg to about 150 mg free base equivalent (FBE).
78. The method of **item** 77, wherein the patient has elevated plasma lyso-Gb₃ prior to initiating administration of the migalastat or salt thereof.
79. The method of **item** 77 or 78, wherein the patient has a mutation in α-Gal A selected from the group consisting of I253T, P259R, G183D, L243F, C174R, D55V/Q57L, G144V, R301Q, G373S, D322E, G325R and Y216C.
80. The method of any one of - **items** 77-79, wherein the migalastat or salt thereof enhances α-galactosidase A activity.
81. The method of any one of i **items** 77-80, wherein the effective amount is about 123 mg FBE.
82. The method of any one of i **items** 77-81, wherein the effective amount is about 123 mg of migalastat free base.
83. The method of any one of i **items** 77-81, wherein the salt of migalastat is migalastat hydrochloride.
84. The method of **item** 83, wherein the effective amount is about 150 mg of migalastat hydrochloride.
85. The method of any one of **items** 77-84, wherein the migalastat or salt thereof is in an oral dosage form.
86. The method of **item** 85, wherein the oral dosage form comprises a tablet, a capsule or a solution.
87. The method of any one of **items** 77-86, wherein the migalastat or salt thereof is administered for at least 6 months.
88. The method of any one of **items** 77-87, wherein administration of the migalastat or salt thereof to a group of classic Fabry patients provides an average decrease in plasma lyso-Gb₃ of at least about 15 nmol/L after 24 months of the administration of the migalastat or salt thereof.
89. A method of treating classic Fabry disease in a patient in need thereof, the method comprising administering to the patient an effective amount of migalastat or salt thereof at a frequency of once every other day, wherein the effective amount is about 100 mg to about 150 mg free base equivalent (FBE) and wherein administering the migalastat or salt thereof reduces plasma globotriaosylsphingosine (lyso-Gb₃) in the patient.
90. The method of **item** 89, wherein the patient has elevated plasma lyso-Gb₃ prior to initiating administration of the migalastat or salt thereof.
91. The method of **item** 89 or 90, wherein the patient has a mutation in α-Gal A selected from the group consisting of I253T, P259R, G183D, L243F, C174R, D55V/Q57L, G144V, R301Q, G373S, D322E, G325R and Y216C.
92. The method of any one of **items** 89-91, wherein the migalastat or salt thereof enhances α-galactosidase A activity.
93. The method of any one of **items** 89-92, wherein the effective amount is about 123 mg FBE.
94. The method of any one of **items** 89-93, wherein the effective amount is about 123 mg of migalastat free base.
95. The method of any one of **items** 89-93, wherein the salt of migalastat is migalastat hydrochloride.
96. The method of **item** 95, wherein the effective amount is about 150 mg of migalastat hydrochloride.
97. The method of any one of **items** 89-96, wherein the migalastat or salt thereof is in an oral dosage form.
98. The method of **item** 97, wherein the oral dosage form comprises a tablet, a capsule or a solution.
99. The method of any one of **items** 89-98, wherein the migalastat or salt thereof is administered for at least 6 months.
100. The method of any one of **items** 89-99, wherein administration of the migalastat or salt thereof to a group of classic Fabry patients provides an average decrease in plasma lyso-Gb₃ of at least about 15 nmol/L after 24 months of the administration of the migalastat or salt thereof.
101. A method of treating gastrointestinal symptoms in a patient having classic Fabry disease, the method comprising administering to the patient an effective amount of migalastat or salt thereof at a frequency of once every other day for treating the gastrointestinal symptoms, wherein the effective amount is about 100 mg to about 150 mg free base equivalent (FBE).
102. The method of **item** 101, wherein the patient has diarrhea prior to initiating administration of the migalastat or salt thereof.
103. The method of **item** 101 or 102, wherein treating one or more gastrointestinal symptoms in the patient comprises reducing symptoms of diarrhea.
104. The method of any one of **items** 101-103, wherein the patient has a mutation in α-galactosidase A selected from the group consisting of I253T, P259R, G183D, L243F, C174R, D55V/Q57L, G144V, R301Q, G373S, D322E, G325R and Y216C.
105. The method of any one of **items** 101-104, wherein the migalastat or salt thereof enhances α-galactosidase A activity.
106. The method of any one of **items** 101-105, wherein the effective amount is about 123 mg FBE.
107. The method of any one of **items** 101-106, wherein the effective amount is about 123 mg of migalastat free base.
108. The method of any one of **items** 101-106, wherein the salt of migalastat is migalastat hydrochloride.
109. The method of **item** 108, wherein the effective amount is about 150 mg of migalastat hydrochloride.
110. The method of any one of **items** 101-109, wherein the migalastat or salt thereof is in an oral dosage form.
111. The method of **item** 110, wherein the oral dosage form comprises a tablet, a capsule or a solution.
112. The method of any one of **items** 101-111, wherein the migalastat or salt thereof is administered for at least 6 months.
113. The method of any one of **items** 101-112, wherein administration of the migalastat or salt thereof to a group of classic Fabry patients provides an average decrease in Gastrointestinal Symptoms Rating Scale for Diarrhea (GSRS-D) of at least about 0.5 after 24 months of the administration of the migalastat or salt thereof.
114. A method of treating classic Fabry disease in a patient in need thereof, the method comprising administering to the patient an effective amount of migalastat or salt thereof at a frequency of once every other day, wherein the effective amount is about 100 mg to about 150 mg free base equivalent (FBE) and wherein administering the migalastat or salt thereof treats one or more gastrointestinal symptoms in the patient.
115. The method of **item** 114, wherein the patient has diarrhea prior to initiating administration of the migalastat or salt thereof.
116. The method of **item** 114 or 115, wherein treating one or more gastrointestinal symptoms in the patient comprises reducing symptoms of diarrhea.
117. The method of any one of **items** 114-116, wherein the patient has a mutation in α-galactosidase A selected from the group consisting of I253T, P259R, G183D, L243F, C174R, D55V/Q57L, G144V, R301Q, G373S, D322E, G325R and Y216C.
118. The method of any one of **items** 114-117, wherein the migalastat or salt thereof enhances α-galactosidase A activity.
119. The method of any one of **items** 114-118, wherein the effective amount is about 123 mg FBE.
120. The method of any one of **items** 114-119, wherein the effective amount is about 123 mg of migalastat free base.
121. The method of any one of **items** 114-119, wherein the salt of migalastat is migalastat hydrochloride.
122. The method of **item** 121, wherein the effective amount is about 150 mg of migalastat hydrochloride.
123. The method of any one of **items** 114-122, **wherein** the migalastat or salt thereof is in an oral dosage form.
124. The method of **item** 123, wherein the oral dosage form comprises a tablet, a capsule or a solution.
125. The method of any one of **items** 114-124, wherein the migalastat or salt thereof is administered for at least 6 months.
126. The method of any one of **items** 114-125, wherein administration of the migalastat or salt thereof to a group of classic Fabry patients provides an average decrease in Gastrointestinal Symptoms Rating Scale for Diarrhea (GSRS-D) of at least about 0.5 after 24 months of the administration of the migalastat or salt thereof.

## Claims

1. Migalastat or salt thereof for use in a method of treating gastrointestinal symptoms in a patient having classic Fabry disease, the method comprising administering to the patient an effective amount of the migalastat or salt thereof at a frequency of once every other day for treating the gastrointestinal symptoms, wherein the effective amount is 100 mg to 150 mg free base equivalent (FBE).

2. Migalastat or salt thereof for use according to claim 1, wherein the patient has diarrhea prior to initiating administration of the migalastat or salt thereof.

3. Migalastat or salt thereof for use according to claim 1 or 2, wherein treating one or more gastrointestinal symptoms in the patient comprises reducing symptoms of diarrhea.

4. Migalastat or salt thereof for use according to any one of claims 1-3, wherein the patient has a mutation in α-galactosidase A selected from the group consisting of I253T, P259R, G183D, L243F, C174R, D55V/Q57L, G144V, R301Q, G373S, D322E, G325R and Y216C.

5. Migalastat or salt thereof for use according to any one of claims 1-4, wherein the migalastat or salt thereof enhances α-galactosidase A activity.

6. Migalastat or salt thereof for use according to any one of claims 1-5, wherein the effective amount is about 123 mg FBE.

7. Migalastat or salt thereof for use according to any one of claims 1-6, wherein the effective amount is about 123 mg of migalastat free base.

8. Migalastat or salt thereof for use according to any one of claims 1-6, wherein the salt of migalastat is migalastat hydrochloride.

9. Migalastat or salt thereof for use according to claim 8, wherein the effective amount is about 150 mg of migalastat hydrochloride.

10. Migalastat or salt thereof for use according to any one of claims 1-9, wherein the migalastat or salt thereof is in an oral dosage form.

11. Migalastat or salt thereof for use according to claim 10, wherein the oral dosage form comprises a tablet, a capsule or a solution.

12. Migalastat or salt thereof for use according to any one of claims 1-11, wherein the migalastat or salt thereof is administered for at least 6 months.

13. Migalastat or salt thereof for use according to any one of claims 1-12, wherein administration of the migalastat or salt thereof to a group of classic Fabry patients provides an average decrease in Gastrointestinal Symptoms Rating Scale for Diarrhea (GSRS-D) of at least about 0.5 after 24 months of the administration of the migalastat or salt thereof.
